# EUROPEAN PATENT SPECIFICATION

(11) **EP 2 819 720 B1**
(45) Date of publication and mention of the grant of the patent: **25.05.2016**
(21) Application number: 13706570.2
(22) Date of filing: 01.03.2013
(51) Int. Cl.: A61M 1/36

(54) **KIT FOR ORGAN PERFUSION**
KIT ZUR ORGANPERFUSION
TROUSSE POUR PERFUSION D'ORGANE

(30) Priority: 01.03.2012 EP 12157765
(43) Date of publication of application: 07.01.2015
(73) Proprietor: Medical Device Works NV, 1070 Brussel (BE)
(72) Inventor: FIERENS, Joop, B-1653 Dworp (BE); NACKARD, Kevin Jason, Flagstaff, Arizona 86001 (US); MARCOUX, Eric Thierry Jean, B-1780 Wemmel (BE); BARTHOLOME, Emmanuel J., B-1180 Uccle (BE)
(74) Representative: Brants, Johan P.E.
(86) International application number: PCT/EP2013/054210
(87) International publication number: WO 2013/128016

(56) References cited:
- US-A1- 2009 112 184
- US-A1- 2011 282 274

## Description

### Field of the invention

The present invention generally relates to the field of medical treatment systems. More particularly, the present invention relates to system, devices, kits and methods to deliver a therapeutic agent and remove its excess during an interventional procedure.

### Background

Embolization, chemo-embolization and radio-embolization therapy are often clinically used to treat a range of diseases, such as hypervascular liver tumors, uterine fibroids, secondary cancer metastasis in the liver, pre-operative treatment of hypervascular menangiomas in the brain and bronchial artery embolization for hemoptysis. An embolizing agent may be embodied in different forms, such as beads, liquid, foam, or glue placed into an arterial vasculature or any combination thereof. The beads may be uncoated or coated. Where the beads are coated, the coating may be a chemotherapy agent, a radiation agent or other therapeutic agent. When it is desirable to embolize a small blood vessel, small bead sizes (e.g., 20 µm - 100 µm) are used. When a larger vessel is to be embolized a larger bead size (e.g., 100 µm - 900 µm) is typically chosen. Embolizing agent therapies are considered minimally or limited invasive therapies and have often provided good results.

Radioembolization (RE) is a well-recognized treatment of primary and secondary liver tumors. It consists in selectively injecting ⁹⁰Yttrium-coupled beads (⁹⁰Y-Beads) in the common hepatic artery or one of its branches. The beads are to embolize and stop in the tumor capillary bed. Within a few days, the beta-radiation emitted by the ⁹⁰Yttirum will kill the tumor cells. A common issue with RE is extrahepatic embolization to the gastric artery or the lungs. Non-targeted delivery of the embolic agent may have significant unwanted effects on the human body. For example, in liver treatment, a number of microspheres can inadvertently reach other non-targeted organs in the body. This has undesirable impacts on other organs including the stomach, the gall bladder, the pancreas and the small intestine and can cause inflammation of the gall bladder (cholecystitis), stomach (gastritis) or intestine (duodenitis). In uterine fibroid treatment, the non-targeted delivery of the embolic agent may embolize one or both ovaries leading to loss of menstrual cycle, subtle ovarian damage that may reduce fertility, early onset of menopause and in some cases substantial damage to the ovaries. Other unintended adverse events including pain and necrosis might appear. These complications require additional medical treatment and might be lethal for the patient which is an enormous disadvantage of RE.

In practice, to avoid these potentially severe side-effects, a simulation is performed before RE, using ^{99m}Technetium labeled microaggregates of albumin (MAA). If the paradoxal embolization (shunting) to the lungs is greater than 20 %, the RE may not take place. If the shunt is between 10 and 20 %, the dose must be reduced with a subsequent decrease of efficacy. The full-dose RE may only be performed if the shunt is less than 10%. For colorectal cancer liver metastasis, at least 20% of the patients may not benefit from RE due to the presence of an intolerable shunt to the lung and up to 38% of the patients may require a reduced dose (which may require an additional, costly treatment). The percentages are even higher for primary liver cancers.

WO 2011/05618 discloses a system for the in-situ treatment of liver disease using local delivery of therapeutic agents. A liver is supplied with therapeutic agents through tubing leading to a catheter located in a hepatic artery. Hepatic venous blood containing concentrations of therapeutic agent (i.e., contaminated blood) is passed via hepatic veins to a double occlusion device catheter located in the inferior vena cava. The occlusion sections of the double occlusion device catheter are positioned central and peripheral of the hepatic veins. The contaminated blood is passed through the double occlusion device catheter to tubing to a point exterior to the body. The contaminated blood is transported through tubing into a blood purification device to detoxify the blood. The detoxified blood is passed through tubing to effect infusion through the subclavian vein. The use of occlusion devices in this system is disadvantageous as the systemic blood flow is interrupted during the treatment, which may not be well tolerated by the patient and can cause hypovolemia. Another drawback of the system of WO 2011/05618 is the use of balloon type devices to occlude the vena cava. These types of devices are highly prone to leakages and migration/rotation.

Moreover, the use of the known infusion/perfusion catheters for the delivery of a therapeutic agent to an organ presents some disadvantages. When positioned in the artery or the vessel in front of an organ such as the lung, kidney or liver, the therapeutic agent will go with the (blood) flow - loading the organ. The non-bonded or uncaptured therapeutic agent will be drained into the system through the organ outflow, which might be the veins or collateral connections. For treatment with a specific therapeutic agent the "go-with-the-flow" situation is less effective as the therapeutic agent will be diluted by the systemic blood flow and will mix with the blood through the organ under normal or slightly reduced flow and will then be conveyed to the patient's systemic circulation.

The present invention provides a solution to at least part of the above mentioned problem by providing a kit as appended by the claims and the description. The present invention allows for the removal of the excess therapeutic agent during RE. Thus, a higher percentage, or even every patient, can benefit from a RE treatment with the appropriate dose of therapeutic agent.

Documents US2009/0112184 A1 and US2011/0282274 A1 disclose further kits according to the prior art.

### Summary of the invention

The present invention provides a kit such as defined in independent claim 1.

The present invention allows the delivery of therapeutic agents to the blood inflow of an organ and the removal of the therapeutic agent excess while maintaining the systemic blood flow of the patient as no occlusion of main systemic arteries and/or veins is required.

The therapeutic agent can be a treatment fluid and/or can be contained in particles also called beads. In a preferred embodiment of the present invention, the particles are beads comprising a radioactive element, preferably polymer or glass beads. In a further preferred embodiment, the size of the particles is comprised between 10 and 300 micrometers, preferably between 15 and 200 micrometers, more preferably between 20 and 60 micrometers, most preferably the particles size is around 30 micrometers.

In a preferred embodiment of the present invention, the tubular member of the second medical device comprises a liquid permeable carrier configured to adopt an essentially cylindrical state when compressed, and to self-expand radially to form said tubular member. In a further preferred embodiment, the carrier is made of a braided wire mesh. The annular ridges are suitable to contact the vessel of the organ thereby apply a sealing pressure to the vessel wall. Hence, the systemic blood flow of the patient is maintained.

The proximal end of the self-expanding tubular member is attached to the distal end of the catheter; said tubular member is configured to expand radially to form a central part flanked by two annular ridges, a distal annular ridge and a proximal annular ridge; the tubular member comprises a liquid-impermeable area disposed with one or more fluid ports for the collection of the organ outflow and two liquid-permeable regions, one distal to the distal annular ridge and one proximal to the proximal annular ridge so forming a passageway between the distal end and the proximal end of the tubular member for the flow the organ blood devoid of therapeutic agent, whereby the liquid-impermeable area is disposed in an area defined by the region flanked by the annular ridges, the distal annular ridge and at least part of the proximal annular ridge for isolating the organ outflow containing therapeutic agent excess from the organ blood flow devoid of therapeutic agent.

In a preferred embodiment, the proximal end of the self-expanding tubular member is attached to the distal end of the catheter; said tubular member is configured to expand radially and comprises a liquid permeable carrier forming a passageway between the distal end and the proximal end of the tubular member for the flow the organ blood devoid of therapeutic agent, said tubular member comprises a liquid-impermeable area having a spoon shape for the collection of the organ outflow, said spoon shaped impermeable area is at least partially attached to the carrier thereby forming an anuular lumen.

In a preferred embodiment of the present invention, the tubular member of the second medical device comprises a liner attached to at least a part of the wall of the tubular member, which provides the liquid impermeable area. This area allows the isolation of the blood flowing in a vessel from blood arising from lateral veins for instance.

In a preferred embodiment of the present invention, the catheter of the second medical device comprises an inner tube, an outer tube surrounding at least a portion of the length of the inner tube and a pusher means for the deployment of the tubular member; the distal end of the pusher means is attached to the proximal end of the tubular member.

In a preferred embodiment of the present invention, the inner tube distal end of the catheter of the second medical device is in fluid connection with the one or more ports of the liquid-impermeable area of the tubular member and the inner tube proximal end is in fluid connection with the separation device inlet.

In an embodiment not forming part of the invention such as claimed, the separation device is an extracorporeal device.

According to the present invention, the separation device is located in the second retrievable medical device.

In a preferred embodiment of the present invention, the separation device comprises at least one filter provided with openings which size is greater than 15 micrometers, preferably between 20 and 30 micrometers, more preferably around 25 micrometers. The filter allows the separation of the therapeutic agent from the blood.

In a preferred embodiment of the present invention, the filter of the separation device is at least partly made of a radioactive resistant material for temporarily storing the particles.

In a preferred embodiment, the kit of the present invention comprises at least one pump suitable to be connected to the proximal end of the second retrievable medical device catheter. The kit migh further comprise at least one blood oxygenator suitable to be connected to the separation device outlet.

In a preferred embodiment of the invention, for organs like liver and lungs wherein retrograde infusion is possible, the second medical device is used for both delivering the therapeutic agent and removing its excess from an organ. The therapeutic agent could be delivered through the outflow of the organ, thereby loading it with a retrograde flow (against systemic pressure, assuring that it will stay in the organ). After some time the flow can be restored again and this will push the non-bonded therapeutic agent out of the organ and can be conveyed by the VCD catheter.

In a preferred embodiment, the kit according of the present invention comprises at least one third retrievable medical device for the isolation and/or the perfusion of veins, said third retrievable medical device comprises a lumen, an inflation lumen, a balloon at the distal end of the device and a plurality of outlets, said outlets are positioned at the distal end of the balloon or at the proximal end of the balloon.

In a preferred embodiment, the present invention provides for the use of the second retrievable medical device for the repair of vessel's lesions.

In a second aspect, the present invention relates to a method for the delivery a therapeutic agent to an organ blood flow and the removal of the excess of said therapeutic agent from said organ blood flow. The method comprises the steps of (a) introducing a first medical device in the organ artery or organ inflow vessel, (b) introducing a second medical device in the organ vein or the organ outflow vessel, without obstruction of the systemic blood flow, (c) controlling the infusion and/or the perfusion flow, (d) injecting the therapeutic agent into the organ artery or the organ inflow using the first medical device, (e) collecting the organ vein blood to the organ outflow having a therapeutic agent excess using the second medical device, (f) circulating the drug loaded perfusate, (g) separating the therapeutic agent excess from the collected organ vein blood or organ outflow using the separation device.

Preferably, the method further comprises the step of redirecting the filtered blood into the organ blood inflow.

In a third aspect, the present invention is also related to the use of the kit as described above and the method as described above for the delivery a therapeutic agent to an organ blood flow and for the removal of the excess of said therapeutic agent from said organ blood flow.

The present invention allows collecting blood from an organ outflow and filtering the collected blood from any therapeutic and/or embolizing agent. The filtering can be performed in the vessel itself such as with the kit according to the invention such as claimed, or in an external separation device to which the collected blood is directed, which does not form part of the invention such as claimed. After filtering, the blood is returned from the external filter to the organ blood flow or systemic blood flow. The kit of the present allows the delivery of high dose drug regime in a local, isolated, environment. The devices of the kit can be placed percutanously, thereby reducing operative trauma and shortening the operation procedure and the hospital stay or completely avoids a hospital stay.

### Description of the figures

Further features, advantages and objects of the present invention will become apparent for the skilled person when reading the following detailed description of embodiments of the present invention, when taken in conjunction with the figures of the enclosed drawings.
**Fig. 1** illustrates a second medical device in the expanded state comprising a tubular member (dumb-bell shaped) attached to a catheter. **FIG. 1A** shows a transverse cross-section across the catheter where the pusher means is a pusher rod.
**FIG. 1B** shows a transverse cross-section across the catheter where the pusher means is formed from the wall of the inner tube.
**Fig. 1C** illustrates another second medical device in the expanded state comprising a tubular member (dumb-bell shaped) attached to a catheter. The liner is attached to the inner wall of the tubular member.
**Fig. 1D** illustrates a side view of another second medical device wherein the distal end of the inner tube have a cup or a spoon shape **Fig. 1E** illustrates a top view of the same embodiment **Fig. 1F** illustrates a cross-section view along A-A shown in **Fig. 1D****.**
**Fig 1G** illustrates another second medical device in the expanded state wherein the device has a bell shape.
**Fig. 2A** illustrates an embodiment of the second medical device of **Fig. 1** where the tubular member is in its collapsed, compressed state and is provided with a closed tip.
**Fig. 2B** illustrates another embodiment of the second medical device of **Fig. 1** where the tubular member is in its collapsed, compressed state and is provided with a conical closed tip.
**Fig. 3** illustrates the second medical device placed in situ, wherein: **A** illustrates a liner on the exterior of the carrier, and **B** illustrates a liner on the interior of the carrier.

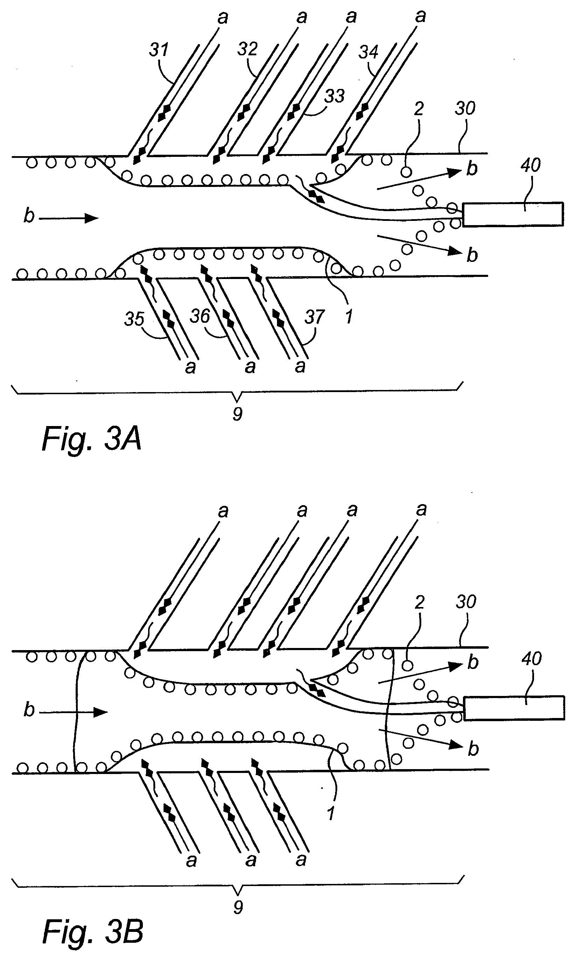

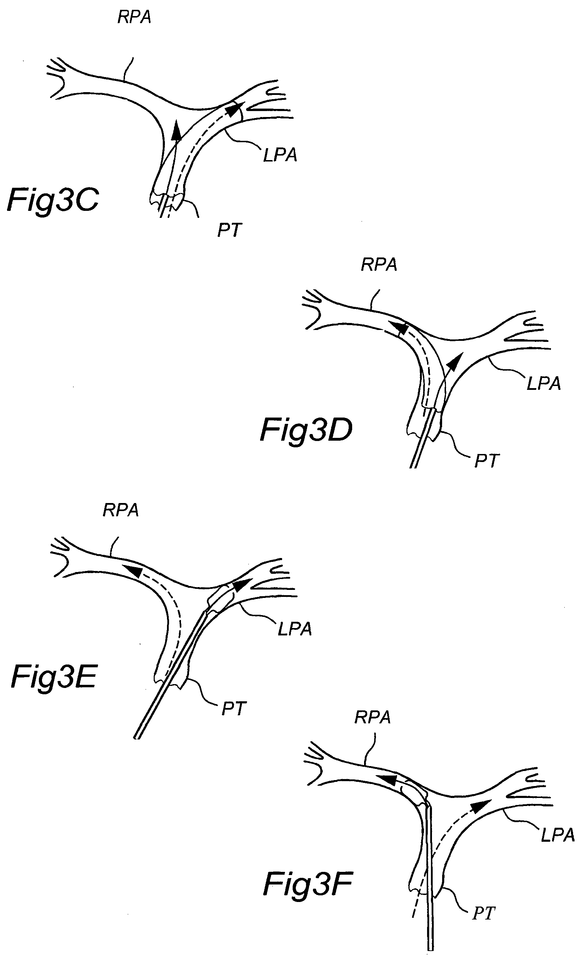

**Fig. 3** **C** and **D** illustrate the use of the second medical device for the delivery of a therapeutic agent to the right and left lung respectively. **Fig. 3** **E** and **F** illustrate the use of the second medical device having a bell shape for the delivery of a therapeutic agent to the right and left lung respectively.

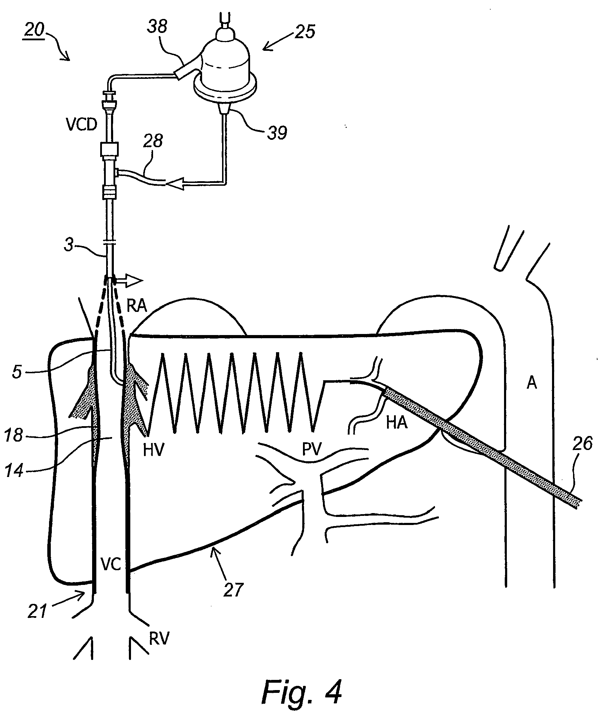

**Fig. 4** illustrates an embodiment of the kit wherein the first medical device, the second medical device and the separation device are used for delivering a therapeutic agent and removing the therapeutic agent excess from the liver, the kit not forming part of the invention such as claimed

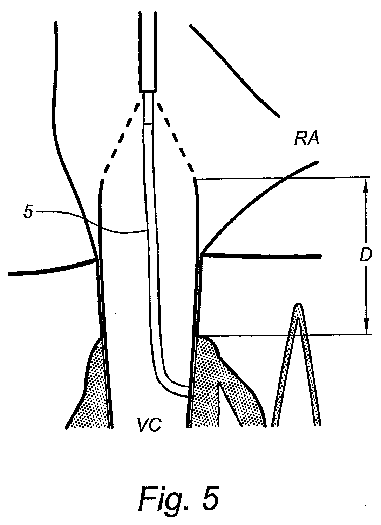

**Fig. 5** illustrates the second medical device when introduced in the vena cava.

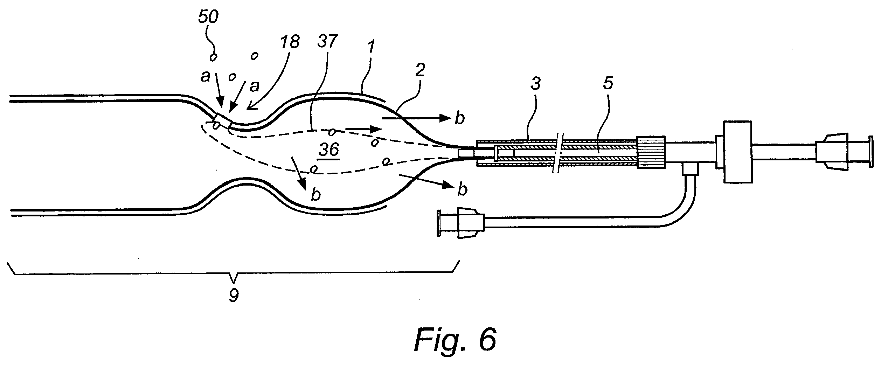

**Fig. 6** illustrates an embodiment showing the position of the separation device within the second medical device which embodiment is according to the invention such as claimed.

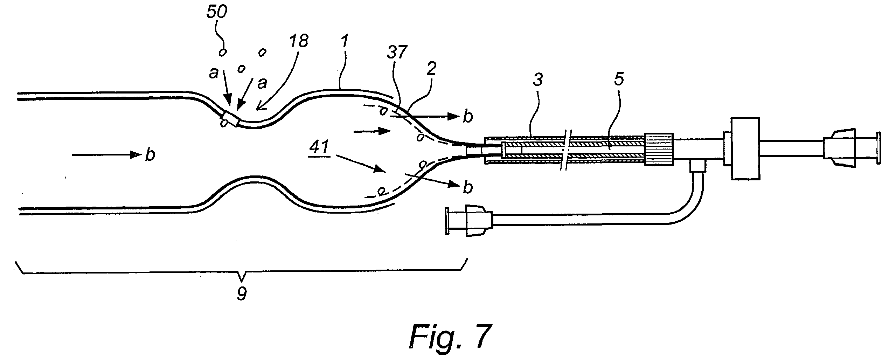

**Fig. 7** illustrates another embodiment showing the position of the separation device within the second medical device, which embodiment is according to the invention such as claimed.

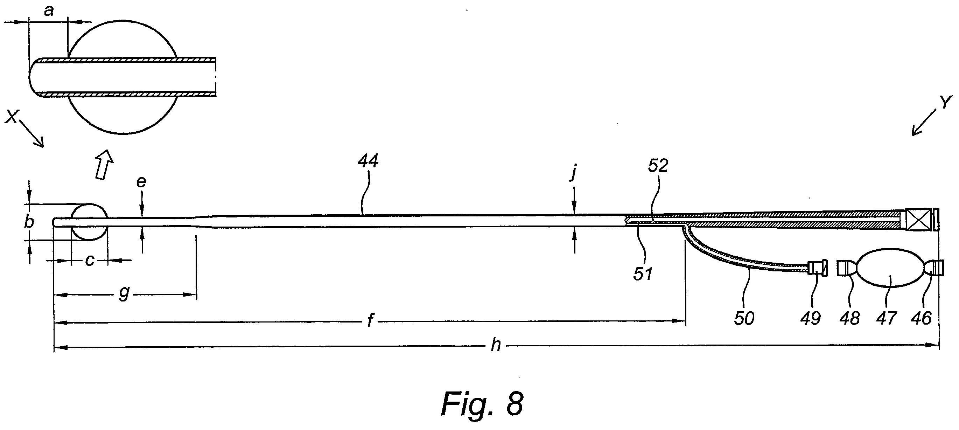

**Fig. 8** detailed schematic illustration of the first medical device.

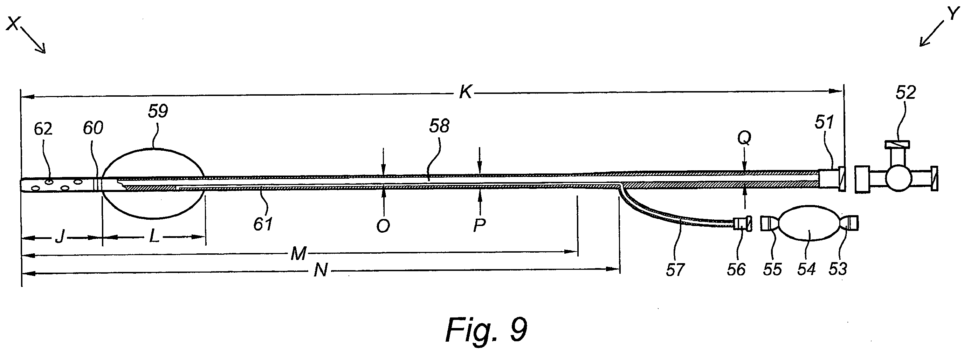

**Fig. 9** detailed schematic illustration of an embodiment of the third medical device.

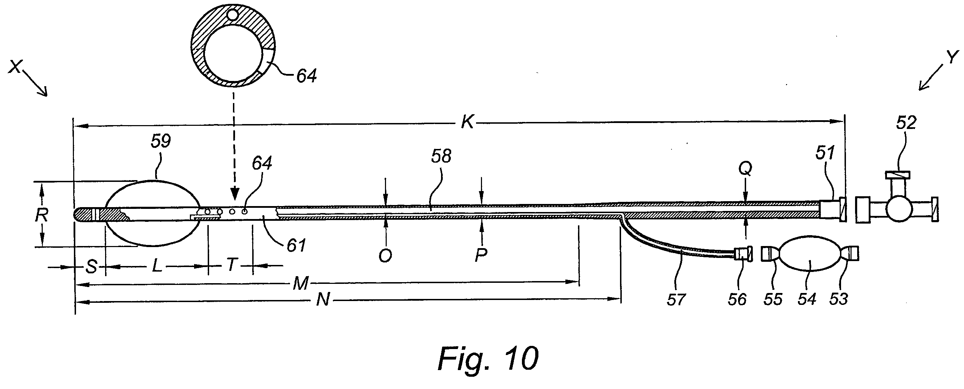

**Fig. 10** detailed schematic illustration of another embodiment of the third medical device.

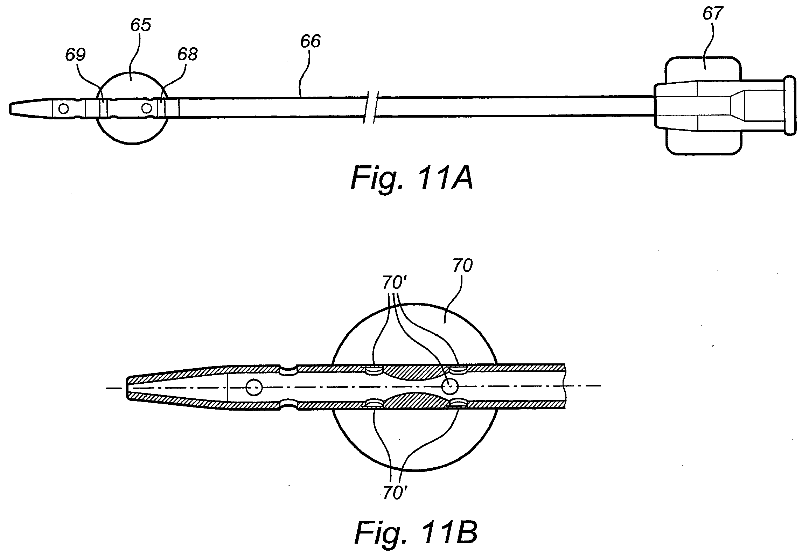

**Fig. 11A** and **Fig. 11B** illustrates an embodiment of the first medical device.

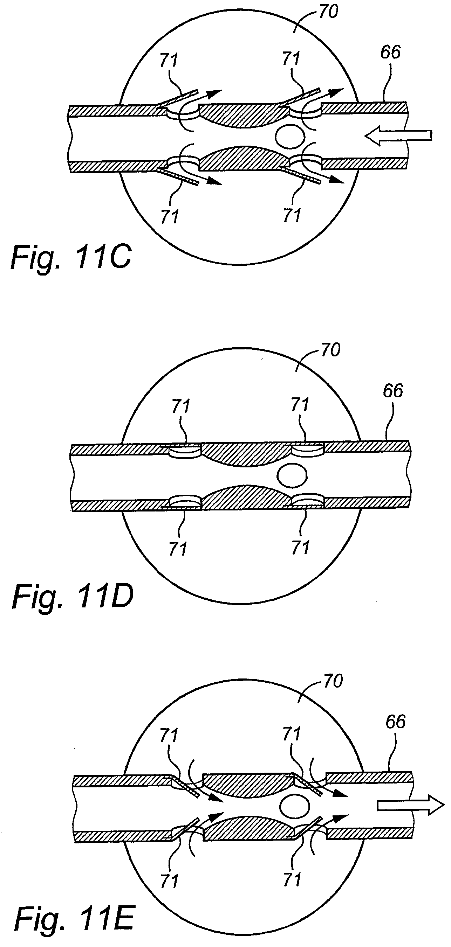

**Fig. 11C**, **Fig. 11D** and **Fig. 11E** illustrate an embodiment of the first medical device wherein each opening allowing fluid communication of the lumen with the interior of the balloon is provided with a valve.

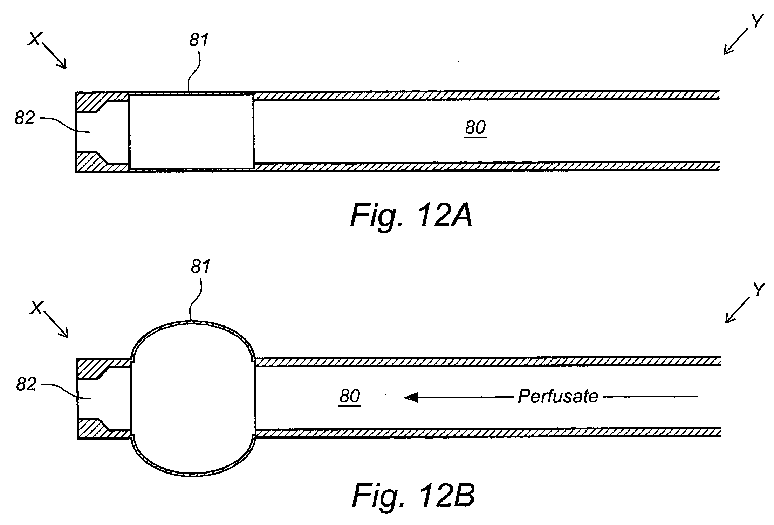

**Fig. 12A** and **Fig. 12B** longitudinal cross-section view of the first medical device

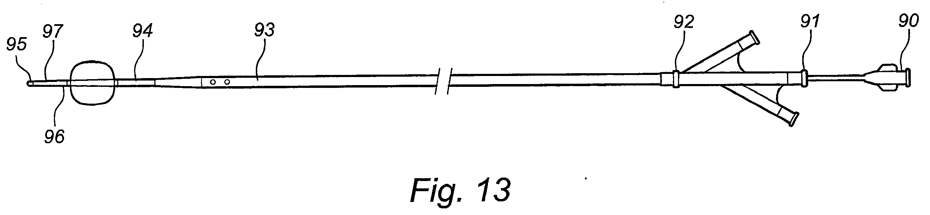

**Fig. 13** illustrates an embodiment of the third medical device.

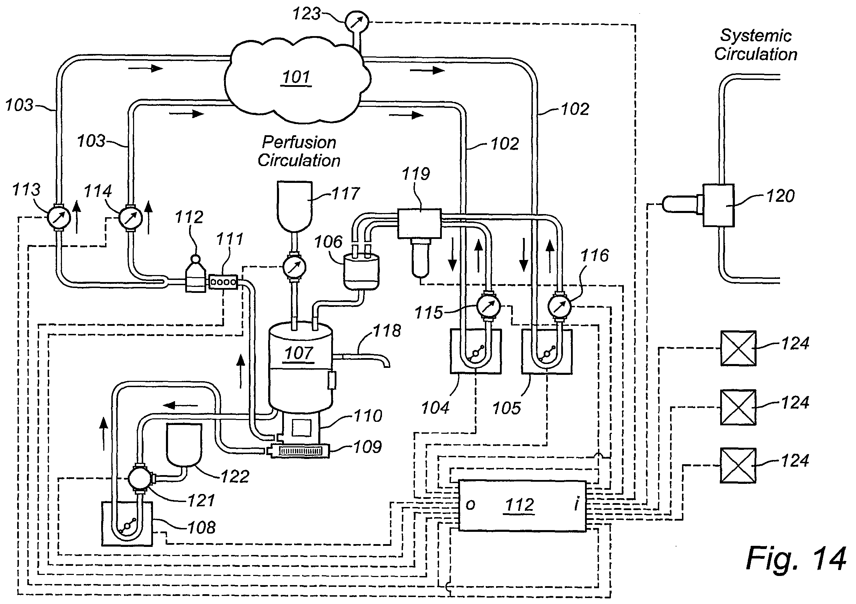

**Fig. 14** is a schematic view of a system that can be used to perfuse a medical treatment through an organ. The system can be controlled by an algorithm.

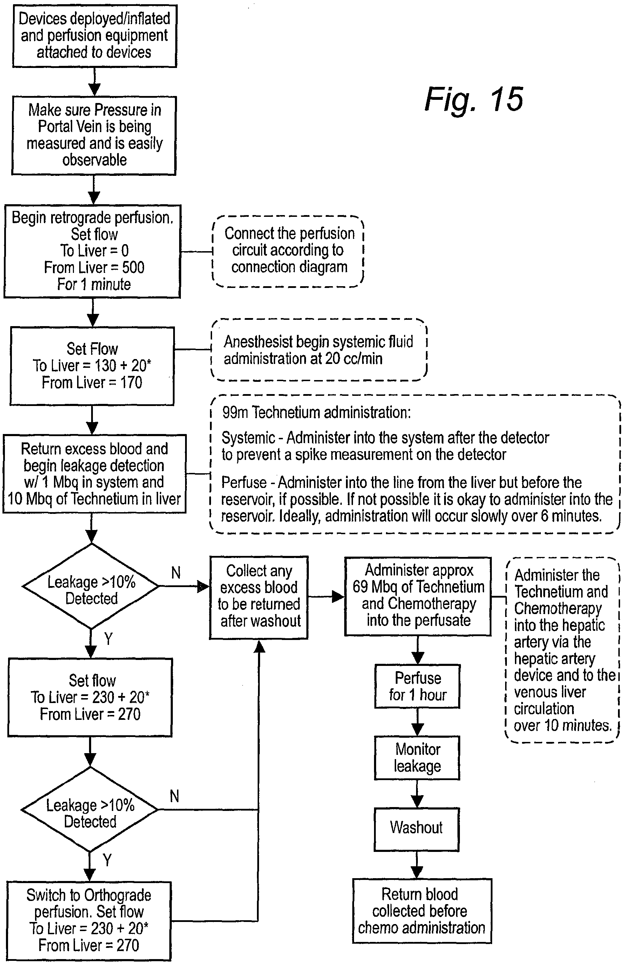

**Fig. 15** is a flowchart of an algorithm that can be used to control the perfusion system of **Fig.14****.**

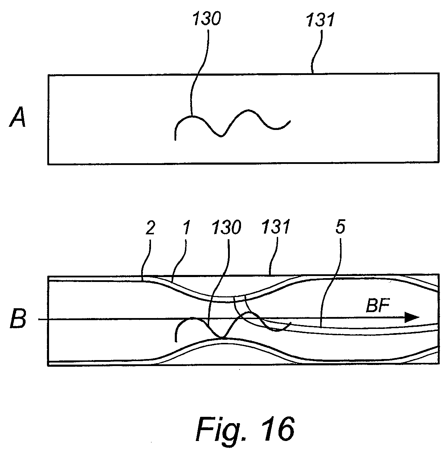

**Fig.** 16A shows a vessel having a lesion **Fig.** 16B shows the second retrievable medical device introduced in the vessel having a lesion.

### Detailed description of the invention

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight" (weight percent), here and throughout the description unless otherwise defined, refers to the relative weight of the respective component based on the overall weight of the formulation.

The term "therapeutic agent" is used herein to refer to a treatment fluid or particles delivered to a patient's organ.
The terms "particles", "microspheres" and "beads" are used herein as synonyms and refer to an object that is substantially spherical in shape and has a diameter less than 1 millimeter. The term "glass" refers to a hard, brittle, non-crystalline, inorganic substance, which is usually transparent; glasses are often made by fusing silicates with soda, as described by Webster's New World Dictionary. Ed. Guralnik, D B 1984.

The terms "inflow" and "outflow" herein refer respectively to the blood flowing inside an organ and the blood flowing outside an organ.

The term "inflow vessel" and "outflow vessel" refer respectively to the vessels directing blood into an organ and the vessels directing blood outside of an organ.

The kit, devices and method of the present invention will be further detailed for a treatment of the liver and/or the lungs. However, any other organ can be treated using the kit, devices and method of the present invention. For liver treatment, the kit of the present invention is intended to control blood flow in and/or out of the hepatic veins, while maintaining continuous blood flow through the inferior vena cava. The kit is used to collect the hepatic outflow in order to filter chemotherapy, particles or a fluid treatment or any other therapeutic agent.

In a first aspect, the present invention provides a kit for the delivery a therapeutic agent to an organ blood flow and the removal of the excess of said therapeutic agent from said organ blood flow, said kit comprises: (a) optionally the therapeutic agent, (b) a first retrievable medical device, for delivering said therapeutic agent into the organ blood flow or organ inflow, preferably into the organ artery blood, comprising a catheter and an injection device such as a syringe, said catheter have a proximal end, a distal end and a lumen; the distal end is in fluid communication with the proximal end via the lumen; said injection device is suitable to be connected to the proximal end of the catheter, (c) a second retrievable medical device for isolating the organ outflow, having a distal end (21) and a proximal end (20); said second medical device comprises a catheter (10) suitable for deploying a self-expanding hollow tubular member (9) ; the proximal end (20) of the tubular member (9) is attached to the distal end (21) of the catheter (10);; the self-expanding member comprises a liquid-impermeable area for the collection of the organ outflow and at least one liquid-permeable region, , and (d) a separation device comprising at least one filter able to separate the therapeutic agent excess from the organ vein blood or organ outflow, having an inlet for the organ vein blood or organ outflow having a particle excess and an outlet for filtered organ vein blood or organ outflow.

### Therapeutic agent

The therapeutic agent of the kit according to the present invention can be a treatment fluid or particles or beads containing said treatment. Particles are known for the person skilled in the art and for instance described in US2004/197264, the content of which is incorporated herein by reference. The particles comprise a material selected from the group consisting of glass, polymer and resin; a first radioisotope that emits a therapeutic [beta]-particle; and a second radioisotope that emits a diagnostic [gamma]-ray; wherein the atomic number of the first radioisotope is not the same as the atomic number of the second radioisotope.

The particles are used to treat organ tumors. The particles are delivered into the organ blood inflow through the inflow vessel of the organ, for instance an artery of the organ to be treated. The radioactive particles are selectively implanted in the microvascular supply of the tumor wherein they become trapped. The particles emit beta radiation for a certain period of time which will kill the tumor.

The particles might be used to treat liver cancer for instance. Patients with primary or metastatic tumors can be treated by radio-embolization via a catheter which tip is placed in the hepatic artery. A direct injection of beads into the tumor is also possible using a needle. The spheres eventually lodge in the microvasculature of the liver and tumor, remaining active until the complete decay of the radioisotope.

The diameter of said particles is in the range from about 1-500 micrometers, preferably 2-400 micrometers, more preferably 4-300 micrometers, most preferably 5-200 micrometers. The diameter of said particles can be any value comprised within the mentioned ranges. Preferably the diameter of said particles is comprises between 50 and 70 micrometers, more preferably between 40 and 60 micrometers, most preferably around 30 micrometers.

### First retrievable medical device

The first medical device **(26,** **FIG. 4****)** of the kit according to the present invention is a device used for the introduction of the therapeutic agent into the patient's body, more in particular into an organ blood flow of the patient. The therapeutic agent may be administered to the patient through the use of syringes or catheters either alone or in combination with vasoconstricting agents or by any other means of administration that effectively causes the microspheres to become embedded in the cancerous or tumor-bearing tissue (U.S. Pat. No. 5,302,369;).

The first retrievable medical device (**26**, **FIG**. **4**) is preferably a catheter. For liver tumor treatment, said catheter will be introduced in the hepatic artery (HA). The insertion of said catheter occurs via the right femoralis artery into the hepatic communis artery.

In a preferred embodiment, the first medical device **(26,** **FIG. 4****)** allows shunt debits in the range of 10 - 500 cc/min allowing slow supply of agents with unwanted tissue reactions, like spasms, and higher flows for bolus treatments.

In a preferred embodiment, the first medical device has a small size and is a flexible device such as it can be positioned following torturous pathways. The diameter of said device is comprised between 5F (= about 1.67 mm) and 7F (= about 2.3 mm). The length of the device is around 800 mm. The latter allows the positioning of the device close to, or in, an organ. The device provides for the control of the blood flow through the targeted organ and provides a non-limited infusion/perfusion debit.

The device is shown in **Fig. 11A** and comprises a grip area **67,** a single lumen **66** and at least one balloon **65.** The expansion of the balloon is induced and controlled by the infusion/perfusion liquid. The device can occlude, at least partly and/or temporarily, the vessel to control the blood flow and to inject a therapeutic agent into that organ in flow rates of at least 20 ml/min. During delivery of the therapeutic agent into the vessel, the infusion/perfusion liquid containing said therapeutic agent flow in the lumen 66 and inflates the balloon 65 by flowing through the openings 68 and 69 **(****FIG. 11** **A**). **Fig. 11B** shows another embodiment of the catheter wherein the openings **70**' allows the flow of the infusion/perfusion liquid leading to the expansion of the balloon **70**.

The device can be further provided with a plurality of valves 71 as shown in **Fig. 11C**, **11D** and **11E****.** Said valves 71 are located on the surface of the openings. When the perfusion liquid is injected a pressure in created in the lumen of the device. Said liquid opens the valves 71 and accumulates in the balloon 70 thereby inflating it as illustrated by the arrows in **Fig. 11C****.** When the injection of the perfusion liquid is terminated the valves 71 close and the balloon remains in an inflated state **(****Fig. 11D****).** When the device is to be retrieved at the end of the treatment, a negative pressure is created inside the lumen of the device which leads to the opening of the valves **71** inside the lumen and to the deflation of the balloon **70 (****Fig. 11E****).** The valves are made of any suitable flexible material such as but not limited to silicon. A pull can be provided in the device to control the opening and closing of the valves.

**Fig. 12A** shows the catheter of the first medical device when the balloon **81** is not in an expanded state. The lumen **80** diameter is reduced at one end **82** of the catheter. The narrowed end can be provided with a conical tip. Reducing the diameter at one end of the catheter leads to a pressure increase during the perfusion and/or delivery of the therapeutic agent. The latter accelerates the expansion of the balloon. The diameter reduction ensures that the balloon segment will expand at minimal defined flows. The diameter at the end **82** of the catheter is reduced by at least 10%, 20%, 30%, 40%, 50%, 60%, 70%, 80%, 90% or any value comprised between these values, compared to the diameter of the lumen to assure the expansion of the balloon during perfusion. **Fig. 12B** shows the catheter when the balloon **81** is in an expanded state due to the flow of the infusion/perfusion liquid in the lumen **80.** Said infusion/perfusion liquid creates a pressure inside the lumen due to the reduced diameter of the catheter end. Said fluid and fluid pressure leads to the inflation of the balloon **81.**

The catheter of the first medical device is made of a biocompatible materials generally applied for short term (< 120 minutes) endovascular procedures. The balloon **81** can be the most flexible part of the catheter, for instance by having smaller wall thickness, or made from other materials bonded to the catheter. The catheter of the first medical device according to the present invention is a percutaneous device having a minimum quantity of material to ensure the vessel occlusion, and to increase the flexibility and maximize the infusion/perfusion flow.

**Fig. 8** shows the catheter having a guide wire **52** and a lumen **51.** The balloon is substantially cylindrical and is positioned at the distal end **X** of the device. The length **c** of the expanded balloon is about 10 mm. The catheter comprises a tube **44** having a lumen. The diameter **j** of said tube **44** is about 2.5 mm. The diameter **e** of distal end **X** portion of the first retrievable medical device is about 2 mm said portion extends over a length **g** of about 150 mm. The length h of the catheter is about 900 mm. The tube **44** is provided at the proximal end **Y** with a female luer adapter **49.** At the distal end **X,** the tube **44** is provided with a balloon that inflates when the user pushes the inflation bladder **47.** The latter is provided with an inflation check valve **46** and a male luer adapter **48.** The inflation bladder **47** is connected to the catheter via a female luer adapter **49** and a connector tube **50.**

### Second retrievable medical device

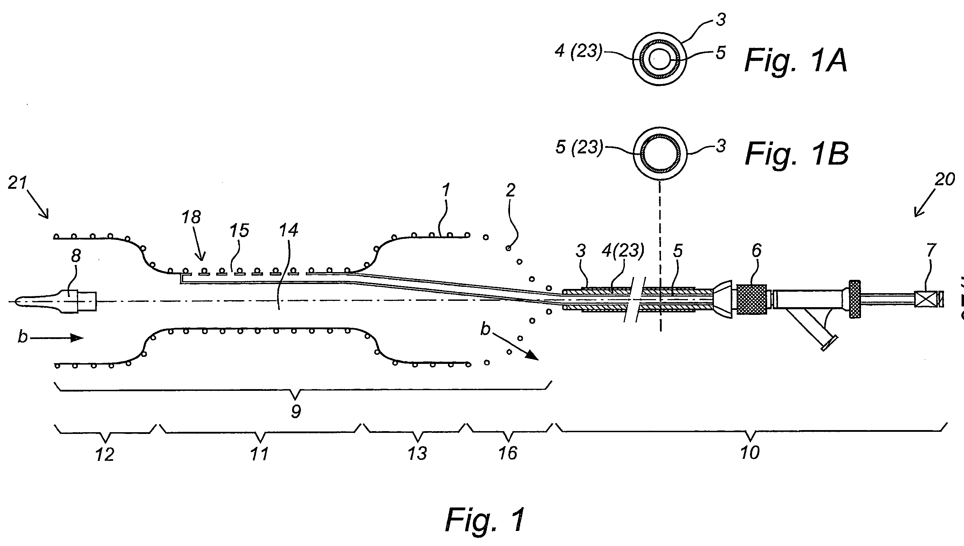

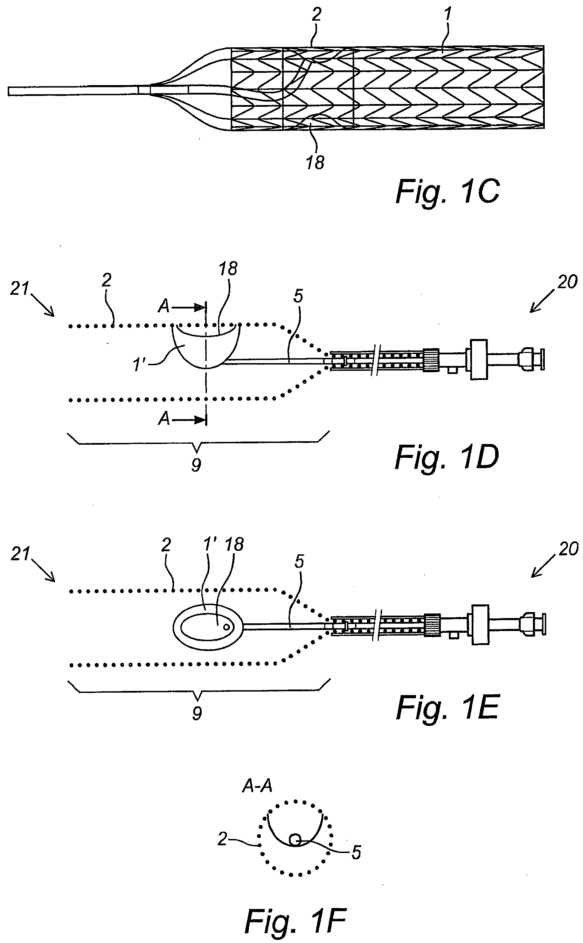

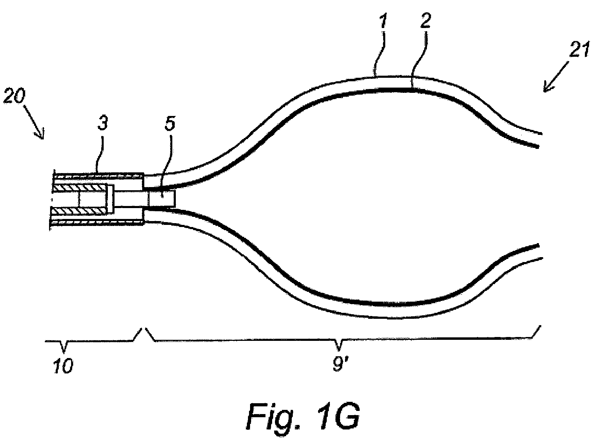

**FIG. 1** illustrates a second medical device The device comprises a radially, self-expandable tubular member **9,** shown in the expanded state attached to a delivery catheter **10.** **FIG 1A** shows a transverse cross-section of the catheter.

The medical device has a distal **21** and a proximal **20** end, comprising a hollow, self-expanding tubular member **9** and a catheter **10** suitable for deploying a self-expanding tubular member **9,** wherein:
- the tubular member **9** is configured to expand radially to form a central part **11** flanked by two annular ridges
- a distal annular ridge **12** and a proximal annular ridge **13,**
- the tubular member **9** comprises a liquid-impermeable area, defined at least by the region flanked by the annular ridges **12, 13,**
- the tubular member **9** comprises two liquid-permeable regions, one distal to the distal annular ridge **12** and one proximal to the proximal annular ridge **13,** so forming a passageway **14** between the distal end **21** and the proximal end **20** of the tubular member **9** for the flow of vessel fluid,
- the proximal end **20** of the tubular member **9** is attached to the distal end **21** of the delivery catheter **10,-** the liquid impermeable area is disposed with one or more fluid ports **15** for blood passage.

The self-expandable tubular member **9,** known herein as 'tubular member', is typically an elastic tube that self-expands after having been compacted. Illustrative examples of self- expandable tubular members are disclosed in the following documents US 5,876,445, US 5,366,504, US 5,234,457, US 5,061,275; Watkinson et al: The role of self-expanding metallic endoprostheses in esophageal structures, "Seminars in Interventional Radiology", 13(1):17-26 (March 1996).

The tubular member **9** comprises, in the expanded state, a central part **11** flanked by two annular ridges - a proximal annular ridge **13** and a distal annular ridge **12.** The central part **11** radially expands to a lesser degree compared to the annular ridges **12, 13.** The expanded central part **11** typically has a cylindrical shape while the annular ridges **12, 13** are at least partly conical, so forming a funnel-like structure in the expanded state. By designing the device as such, it is formed partly as an hour-glass or dumb-bell upon expansion. When deployed in a bodily vessel, the central part **11** forms an annular lumen **18,** sealed by the annular ridges **12, 13** for the collection of blood brought to said bodily vessel by other vessels, such as lateral vessels.

The skilled person will appreciate the diameters of the tubular member **9** at the annular ridges **12, 13** and central part **11** in the expanded state may be adapted according to the diameter of the vessel at the deployment site. The diameter of the central part **11** should be wide enough to avoid obstruction of blood flow, but not too wide that flow reaches high levels that will affect leakage resistance and disturb laminar flow. The diameter of the annular ridges **12, 13** should be selected such as to provide a perfect sealing against the vessel's inner wall when the tubular member **9** is in the expended state. The sealing is assured by the pressure applied by the carrier **2** on the vessel's wall and by the fact that the annular ridges **12, 13** contact the inner vessel over a distance comprised between 15 and 100 mm, preferably between 16 and 80 mm, more preferably between 17 and 60 mm, most preferably between 18 and 40 mm.

In an embodiment not forming part of the invention such as claimed, in the expanded state the central part and the annular ridges of the tubular member are designed to expand radially with the same degree. The expanded tubular member has then a cylindrical shape **(****Fig. 1C**) with at least partly conical extremities, so forming a funnel-like structure in the expanded state. The cylindrical shape is obtained by the expansion of the carrier **2.** A liner **1** having an hour-glass or dumb-bell shape is at least partly attached to the inner walls of the carrier **2** as shown in **Fig. 1C**. When deployed in a bodily vessel, liner **1** forms an annular lumen **18,** for the collection of blood brought to said bodily vessel by other vessels, such as lateral vessels. The straight carrier **2** is made of flexible material such as braided wire mesh. Hence, it is able to follow the vessel curvatures and assure a reliable isolation between the systemic blood flow and the fluid flowing in through both the inner tube **5** and the annular lumen **18.** The continuous cylindrical carrier assures an optimal opening of the vessel during the organ treatment.

In another embodiment not forming part of the invention such as claimed, the proximal end **20** of the self-expanding tubular member **9** is attached to the distal end **21** of the catheter **10.** Said tubular member is configured to expand radially and comprises a liquid permeable carrier **2** forming a passageway between the distal end **21** and the proximal end **20** of the tubular member for the flow the organ blood devoid of therapeutic agent. Said tubular member comprises a liquid-impermeable area having a spoon shape **1'** for the collection of the organ outflow, said spoon shaped impermeable area is attached in at least two locations to the carrier **2** thereby forming an anuular lumen **18.** In the expanded state the central part and the annular ridges of the tubular member are designed to expand radially with the same degree. The expanded tubular member has then a cylindrical shape **(****Fig. 1D** and **Fig. 1E****)** with at least one partly conical extremity, so forming a funnel-like structure in the expanded state. The cylindrical shape is obtained by the expansion of the carrier 2. The distal end 21 of the inner tube **5** is formed as a cup or a spoon **1**'. Preferably, the cup or spoon **1'** of the device is made of an impermeable material, thereby defining a cup or spoon shaped impermeable area. The cup or spoon can be attached in at least two locations to the carrier **2** such as to form an annular lumen **18** as shown in **Fig. 1D****.** Said cup or spoon **1**' can be bonded completely to the carrier **2.** To exemplify the latter, the cup lip, defining the cup upper opening, is completely bonded to the carrier and the inner volume of the cup, wherein fluid can be placed in regular cup, is hanging inside the tubular member as shown in **Fig. 1E** and **Fig. 1F****.**

The cup or a spoon **1'** is suitable to be in fluid connection with several bronchial branches. **Fig. 1F** shows a cross sectional view along A-A shown in **Fig. 1D** while **Fig. 1E** shows a top view of the second medical device according to the present embodiment. The straight carrier **2** is made of flexible material such as braided wire mesh. Hence, it is able to follow the vessel curvatures and assure a reliable isolation between the systemic blood flow and the fluid flowing in through both the inner tube **5** and the annular lumen **18.** The continuous cylindrical carrier assures an optimal opening of the vessel during the organ treatment. The carrier **2** can be devoid of impermeable liner. Said carrier can also be provided with an impermeable liner over at least part of its length or over its full length.

In another embodiment not forming part of the invention such as claimed, the second medical device in expanded state has a bell shape as shown in **Fig. 1G** provided with a cup or spoon as shown in **Fig. 1E** and **Fig. 1F****.** Said cup or spoon is made of an impermeable material, thereby defining a cup or spoon shaped impermeable area. The carrier of the bell shaped member can be bonded to an impermeable liner. Preferably said carrier is devoid of impermeable liner. The cup or spoon can be attached in at least two locations to the carrier **2** such as to form an annular lumen **18** as shown in **Fig. 1D****.** Said cup or spoon **1'** can be bonded completely to the carrier **2.** To exemplify the latter, the cup lip, defining the cup upper opening, is completely bonded to the carrier and the inner volume of the cup, wherein fluid can be placed is hanging inside the tubular member as shown in **Fig. 1E** and **Fig. 1F****.** The example refers to a regular cup for coffee or thee or any other liquid.

Preferably, the cup or spoon **1'** is placed around the inlet or outlet of a side vessel thereby allowing perfusion of said vessel while being isolated from the main flow. This considerably limits side effects to other side vessels. If the perfusion of the bronchial arterial delta is desired, the device allows isolating and perfusion of said delta only. All other arteries leaving the aorta remain open for arterial inflow to prevent serious problems during the local perfusion. The device presents the advantage of being flexible and having an uncovered braided wire mesh carrier so it can be easily placed and anchored in tortuous situations, like the ascending/descending aorta or below, or in the pulmonary arteries at the left/right Y split. The cup or spoon shaped impermeable area will assure that we only communicate, and control the flow, with the targeted vessels.

Using small edging of the cup or spoon of the device of the present invention and placing said edging around the bronchial delta we can inject contrast in the delta, and the radiologist will have visual control of the isolation, thereby allowing optimization of the device position when needed. When the edging of the cup or spoon of the device is optimized for certain side vessels such as the bronchial artery delta, the branching between left or right pulmonary artery or the branching between the internal- and external jugularis; perfusion into/from specific side vessels, without occluding the main flow or interrupting blood flow to other organs in the same area, is then possible.

It is to be understood that the non-permeable area of the device can have any other shape provided said shape is suitable to be positioned in front of one or a group of vessels thereby allowing selective perfusion into selected vessels without communication of the perfusate to other side vessels or to the systemic flow.

In another embodiment not forming part of the invention such as claimed, the second medical device in expanded state has a bell shape as shown in **Fig. 1G****.** The device comprises a carrier **2** and liner **1** which is disposed at the outer wall of the carrier **2.** The liner **1** is in this embodiment provided over the full length of the device as shown in **Fig 1****. G.** The device is mainly used for delivering a therapeutic agent to an organ. The therapeutic agent flows through the inner tube **5.**

It is to be understood that all the embodiments of the second medical device described above can be used for delivering a therapeutic agent to an organ and/or retrieving a therapeutic agent from an organ. The delivery and/or retrieval of the therapeutic agent is performed while maintaining the systemic blood flow of the patient. The treated organ is for example liver or lungs.

The use of the second medical device for the delivery of a therapeutic agent to an organ, in particular the lungs, is illustrated in **Fig. 3** **C** to **F**. After being inserted in the pulmonary artery **(PA)** or in one of the lung segments, the therapeutic agent is delivered through the inner tube **5.** **Fig. 3** **C** illustrates the use of the second medical device for the delivery of a therapeutic agent to the right lung (continued arrow) while the systemic flow (illustrated on the figure by a discontinued arrow) of the left lung is maintained. **Fig. 3** **D** illustrates the use of the second medical device for the delivery of a therapeutic agent to the left lung (continued arrow) while the systemic flow (discontinued arrow) of the right lung is maintained. **Fig. 3** **E** illustrates the use of the second medical device having a bell shape for the delivery of a therapeutic agent to the right lung (continued arrow) while the systemic flow (discontinued arrow) of the left lung is maintained. **Fig. 3** **F** illustrates the use of the second medical device having a bell shape for the delivery of a therapeutic agent to the left lung (continued arrow) while the systemic flow (discontinued arrow) of the right lung is maintained. The outer diameter of the device is about 33 mm to occlude when used in one of the pulmonary arteries and about 10 mm when used in a lung segment. The length of the devices is about 50mm.

The second medical device is positioned in the vena cava with the proximal extremity just above the inflow of the renal veins. The proximal part of the device occludes collateral inflow form renal veins up to the annular lumen **18** around the hepatic veins. This isolation is assured by an annular ridge that is rounded at the end, preventing possible damage of the entrance of the right atrium even when placed deep into the right atrium.

The annular lumen **18** around the hepatic veins is about 15-20 mm long. The practitioner will not encounter difficulties to position the device in front of the hepatic veins. With exception of the annular lumen **18,** the vena cava is in contact with the second medical device: from renal veins up to right atrium.

The minimum diameter of the central part **11** in the expanded state may be 45, 50, 55, 60, 65, 70, 76, 80, 85, 90, or 95% of the internal diameter of the vena cava, or a value between any two of the aforementioned values. Preferably the minimum diameter of the central part **11** in the expanded state is at least 50% of internal diameter of the vena cava. According to one aspect of the invention, the minimum diameter of the central part **11** in the expanded state is between 6, 8, 10, 12, 14, 16, 18, 20, 22 mm or a value in the range between any two of the aforementioned values, preferably 8 to 18 mm diameter.

The maximum diameter of the annular ridges **12,13** in the expanded state may be 5, 10, 15, 20, 25, 30, or 35% larger than the internal diameter of the vena cava, or a value between any two of the aforementioned values. Preferably the maximum diameter of the annular ridges **12,13** in the expanded state is between 10, 15, 20, 25, 30% larger than internal diameter of the vena cava. According to one aspect of the invention, the maximum diameter of the annular ridges 12,13 in the expanded state is between 20, 25, 30, 35, 40, 45 mm or a value in the range between any two of the aforementioned values, preferably 20, 26, 33 or 43 mm diameter.

According to one aspect of the invention, the difference between the maximum diameters of the annular ridges **12, 13** and the minimum diameter of the central part **11** in the expanded state may be 2, 3, 4, 5 or 6 mm or a value in the range between any two of the aforementioned values, preferably 4 to 5 mm diameter.

The region flanked by the annular ridges **12, 13** defines a liquid impermeable area. The skilled person will understand the adaptations to the tubular member **9** necessary to define a liquid-impermeable area that provide sealed annular lumen **18** in the deployed state. Generally, the liquid-impermeable area will extend between the annular ridges **12, 13,** from the region of maximum diameter of the proximal annular ridge **13** to the region of maximum diameter of the distal annular ridge **12.** It is within the practices of the skilled person to determine a lesser or greater area, for example, depending on the patency of the vessel wall.

Through the inner part of the tubular member extends a passageway **14** between the distal end **21** and the proximal end **20** of the tubular member **9;** blood is able to flow there between. The tubular member **9** comprises two liquid-permeable areas, one distal to the distal annular ridge **12** and one proximal to the proximal annular ridge **13,** so blood can flow from through the passageway from the distal end **21** and the proximal end **20** or vice versa. Preferably, the liquid-permeable region of the distal end **21** of the tubular member **9** comprises an open- mouthed region, while the liquid-permeable region of the proximal end **20** comprises a region **16** devoid of liquid-impermeable lining. According to one aspect of the invention the tubular member **9** comprises a carrier **2** and a liquid-impermeable liner **1.** The carrier part **2** is typically, though not always, the outmost part of the device, and contacts the vessel wall in the deployed state. The carrier **2** expands in the manner mentioned above. The carrier **2** is preferably retractable which means that it normally adopts the hour-glass or dumb-bell shape mentioned above; when retracted into a cylindrical sheath, the carrier can be compressed to adopt an essentially cylindrical state, suitable for introduction into and freely positioning within a vessel. The carrier **2** can be described as being self-expanding. The carrier is attached to the catheter, to a pusher means **23** element therein described below. Preferably, the proximal end **20** of the carrier **2** is attached circumferentially to the distal end **21** of the pusher means **23,** so giving the proximal end **20** of the tubular member **9** a conical shape **16.**

The tubular member **9** or carrier **2** is attached to the catheter **10** or pusher means **23.** It is configured to remain attached when the tubular member **9** or carrier **2** is in the retracted and deployed position. According to one aspect of the invention, it is non-releasably attached, meaning that the tubular member **9** or carrier **2** cannot be released, in situ, from the catheter **10** or pusher means **23.** In other words, the device may be devoid of a mechanism for releasing the tubular member in situ. This feature allows the tubular member to be withdrawn at the same time as the catheter **10,** without the possibility of leaving the tubular member in the vessel. A non-releasable attachment may still allow the member **9** or carrier **2,** to be unattached from catheter **10** or pusher means **23** outside by the body, for example, using a screw fitting, a clip, a push fitting or any other secure coupling. A non-releasable attachment would also include the possibility that the member **9** or carrier **2** is permanently attached to the catheter **10** or pusher means **23.**

In the expanded state, the carrier **2** is able to retain its shape without the requirement for an additional source of pressure, for example, from a balloon catheter. The carrier **2** may or may not maintain an essentially constant axial length in the compressed state compared with the expanded state.

The carrier part 2 is preferably made of a braded wire mesh, woven so as to self-expand radially. In an embodiment, the carrier is made from a surgical wire preferably of an alloy comprising Cobalt, Chromium, Nickel, Molybdenum and Iron, and more preferably a surgical wire in accordance to the standard ASTM F 1058. Alternatively, the carrier part **2** may be a knitted mesh of nitinol wire flexible in both the radial and longitudinal axes. Alternatively, other materials, such as shape memory alloy or synthetic material, can be used to produce the carrier. The carrier part **2** may, alternatively, be laser cut. The shape of the central part **11** may be formed by using crimping or heat treatment. The carrier may show a high degree of flexibility and a radial force that assures a good contact with the vessel wall after positioning. The carrier part **2** is liquid permeable, which means that blood can flow there trough, without substantial hindrance. This is achieved in the carrier because it is formed from an open wire structure and may comprise an open mouthed end. A liquid permeable region may comprise one or more openings, at least wide enough to avoid capillary action though the opening.

Because the carrier part **2** is preferably formed from an open mesh structure, it contacts the vessel wall securely in the expanded state, owing to the open structure, creating a plurality of friction points. In an expanded state, the device is securely anchored and provides strong sealing against the vessel wall. There is no requirement for applying additional pressure to the vessel walls, for example, from a balloon.

Another component of the tubular member **9** is the liner **1,** which is made from a liquid-impermeable material. This is typically attached partly to the walls of the carrier **2,** inside or outside the passageway lumen **14** of the device. The liner **1** is disposed at least in the region of the annular lumen **18,** so that the passageway lumen **14** is liquid-sealed from the annular lumen **18** in the deployed state. Preferably the liner **1** is disposed in an area defined at least by the region flanked by the annular ridges **12, 13.**

The liner **1** may be made from a biocompatible material, preferentially a medical grade expandable material e.g. an elastic material which can expand at the same time as the carrier **2.** The liner may be made from a medical grade polycarbonate polyurethane formulation. The liner may, alternatively, be made from ploytetrafluoroethylene, polyurethane, silicone or polyethylene terephthalate polymers. The most preferred materials are indicated in Table 1 below:

**Table 1: Examples of preferred liner materials for use in the present invention. All brand names are registered trademarks.**

| | **Supplier** | **Brand name** | **Elastomer** |
|---|---|---|---|
| 1 | Polymer Technology group Inc, Berkeley CA, USA (Licensed by Boston scientific) | Bionate (Corethane) | Thermoplastic Polycarbonate Urethane |
| 2 | B.F Goodrich, Cleveland OH, USA | Estane | Thermoplastic Polyesther Urethane |
| 3 | Thermedics Inc, Woburn MA, USA | Tecoflex | Thermoplastic PolyetherUrethane |
| 4 | CT Biomaterials, Woburn USA | Chronoflex | Thermoplastic Aromatic Polycarbonate Polyurethane |
| 5 | Aortech, Sidney, Aus | Elasteon | Siloxane based Macrodiol Aromatic Polyurethane |

The liner **1** may be attached to the carrier **2** by chemical or thermal bonding. The liquid-impermeable area formed by the liner **1** is disposed with one or more fluid ports **15** for blood passage; this is described in more detail further below.

Through the inner part of the tubular member **9** extends a passageway **14** between the distal end **21** and the proximal end **20** of the tubular member **9;** blood is able to flow there between. The tubular member **9** comprises at least two liquid-permeable areas, one distal to the distal annular ridge **12** and one proximal to the proximal annular ridge **13,** so fluid can flow from through the passageway **14** from the distal end **21** and the proximal end **20** or vice versa. A flow is indicated by arrows 'b' in **FIG. 1****.** The skilled person will realize that liquid permeable areas should not extend into the liquid impermeable region so that the seal of the annular lumen **18** is breached. Preferably, the distal end **21** of the tubular member is open-mouthed, while the proximal end **20** is closed but is disposed with a liquid-permeable region **16** i.e. a region devoid of liner **1.**

According to one aspect of the invention, a region of the carrier **16** towards the proximal end **20** of the proximal annular ridge **13,** is devoid of liner **1.** According to another aspect of the invention, at least part of the carrier **2** between the distal end **21** of the catheter **10** and proximal end **20** of the proximal annular ridge **13** is devoid of liner **1.** This creates a large liquid passageway **14** inside the tubular member **9** while the catheter **10** is still attached. This configuration has advantages over conventional designs which employ openings and lumens within the narrow confines of the catheter tube to maintain the flow of blood. Conventional lumens are narrow bore, and can cause the buildup of pressure towards the proximal side of the occlusion device. Known devices thus require catheter tubing having larger diameters to accommodate wider a blood lumen, which catheters can then be difficult to navigate along a tortuous path of a blood vessel for example. The present device, by contrast, dispenses with a catheter blood lumen, and maintains blood flow using a wide bore passageway in the expanding tubular member **9,** and with a narrow diameter catheter.

The catheter **10** part of the device is used to introduce and guide the tubular member **9** into the body vessel. The catheter **10** is also used to restrain, temporarily, the tubular member **9** in a compressed state at the distal end of the catheter. It is also used to withdraw liquid to from the annular lumen **18**. In use, the catheter is introduced to a desired site within a body vessel, the restraint is removed, thereby allowing the tubular member **9** to expand by its own elastic and apply sealing pressure to the vessel wall using the annular ridges **12, 13.**

Examples of delivery systems for expandable tubular members are described in the following US patents US 5,484,444, US 4,990,151 , and US 4,732,152.

According to one embodiment the catheter **10** comprises an outer tubing 3, pusher means **23** for deployment of the tubular member **9,** and an inner tube **5,** which extend along the length of the catheter. The pusher means **23** may be a pusher rod (or stick) **4** at least partly co-axially or concentrically disposed around the inner tube **5 (****FIG. 1A****).** Alternatively, the pusher means **23** may be formed from the wall of the inner tube **5 (****FIG. 1B****).**

The outer tubing **3** may be coaxially or concentrically disposed around the pusher rod **4.** Where the pusher means **23** is formed from the wall of the inner tube **5,** the outer tubing **3** may be coaxially or concentrically disposed around the inner tube **5.** The pusher means **23** is configured to translate axially along the length of the catheter, relative to the outer tubing **3.**

Where the pusher means **23** is formed of the wall of the inner tube **5,** the inner tube **5** may be configured to translate axially along the length catheter, relative to the outer tubing **3.** Movement of the pusher means **23** may be effected by operating a plunger **7** mechanically connected to the pusher rod **5** or inner tubing **5,** at the proximal end **20** of the catheter **10.**

The position of the outer tubing **3** may be maintained or adjusted using a grip area **6.** The distal end **21** of pusher means **23** is mechanically attached to the proximal end of the carrier **2.**

According to one embodiment of the invention, the catheter **10** comprises, (a) an inner tube **5;** (b) an outer tube **3,** (c) a pusher means **23.** Said outer tube **3** is surrounding at least a portion of the length of said inner tube **5.** Said pusher rod **4** may be disposed between said inner tube **5** and said outer tube **3.** Alternatively, the pusher means **23** may be formed of the wall of the inner tube **5,** in which case the outer tube **3** surrounds at least a portion of the length of said inner tube **5** whose wall forms the pusher means **23.** The pusher rod **4** is adapted for axial movement relative to said outer tube. The tubular member **9** is attached to the distal end of the pusher means **23,** and may be retracted in the outer tube **3** in the compressed state.

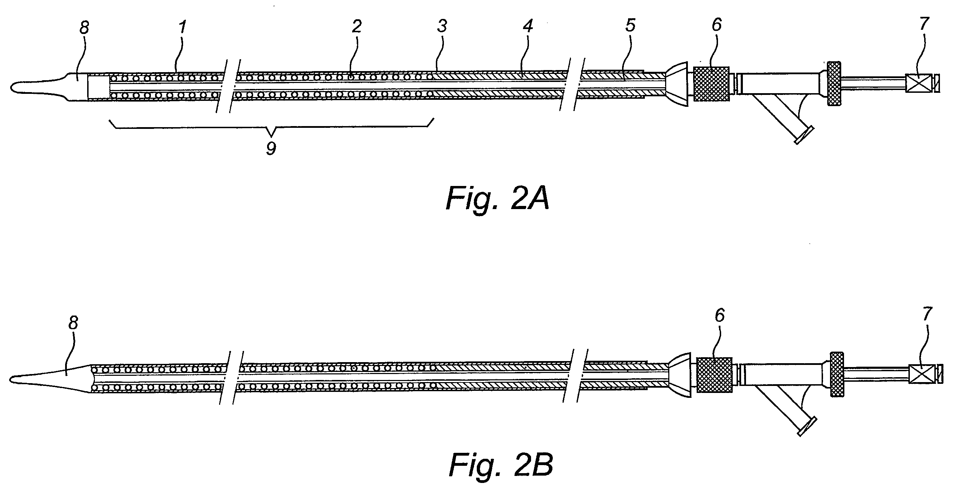

**FIG. 2** illustrates the tubular member **9,** comprising the liner **1** and carrier **2,** retracted within the outer tubing **3** of the catheter **10.** In the compressed state, the tubular member **9** is maintained compressed by inner surface of the outer tube **3,** which acts as a restraint. **FIG. 2A** shows an embodiment wherein a closed tip **8** is provided to the device at the distal end **21.** **FIG. 2B** shows an embodiment wherein a conical closed tip **8'** is provided at the distal end **21** of the device. Said conical closed tip **8'** acts as a dilator which helps opening and enlarging the vein during the introduction of the second medical device.

According to one aspect of the invention, the catheter **10** further comprises a restraining member disposed between said outer tube **3** and tubular member **9,** said restraining member being dimensioned to maintain said tubular member **9** in a compressed state.

The aforementioned restraining member may be a braided tube -or any other type of tube-surrounding said tubular member **9,** said braided tube preferably being made from a strong, flexible, filamentary material having a low coefficient of friction. Examples of such materials may be a fine polyester or metal wire. The braided tube may be formed directly over the tubular member **9,** preferably using an automated braiding machine, or may be pre-formed and then inserted over the tubular member **9.** Where the braided tube is pre-formed and inserted over the tubular member **9,** the system preferably further includes a braid holding sleeve secured to the inner tube **5,** said braid holding sleeve being adapted to receive the proximal end of the braided tube. The distal end of the restraining member is preferably mechanically coupled to the distal end of the outer tube **3** so that retraction of the outer tube **3** causes the restraining member to retract from the tubular member **9,** thereby allowing the tubular member **9** to self-expand. The catheter **10,** and tubular member **9** may be inserted in a blood vessel and, preferably with the aid of a guide wire, manoeuvred to its desired position. The guidewire may be disposed with a separate guidewire lumen. Alternatively, the inner tube **5** may act as a guidewire lumen. The tubular member **9** may be deployed by moving the pusher means **23** axially in the distal direction, while the outer tubing **3** is held in a fixed position. Preferably, the tubular member **9** is deployed by retracting the outer tubing **3** axially in the proximal direction while the pusher means **23** is held in a fixed position. The practitioner may position the tubular member appropriately to account for any shortening of the device during deployment. As the restraint of the outer tubing is removed, the tubular member **9** self-expands. The catheter may optionally be closed with a tip **8.**

As seen in **FIG. 1** the second medical device comprising the liner **1** and carrier **2** expands to its dumb-bell or hour-glass shape. Retrieval of the device after treatment is by withdrawing the tubular member **9** into the outer tubing **3.** This may be achieved by drawing the pusher means **23** towards the proximal end **20** while maintaining the position of the outer tubing **3.** Alternatively, the outer tubing **3** may be pushed towards the distal end **21** while maintaining the position of the pusher rod **4.** Because the carrier **2** is connected to the pusher means **23,** the tubular member **9** is forced to take its non-expanded state inside the outer tubing **3** again. The device can then be carefully withdrawn from the blood vessel.

The inner tube **5** of the catheter **10** is in fluid connection with the one or more ports **15** present in the wall tubular member **9.** Said port **15** is disposed in the wall of the tubular member 9 in the region of the annular lumen **18.** The port **15** may be disposed in the central part **11,** and/or in the parts of the annular ridges **12, 13** that form the annular lumen **18.** The port **15** allows the lumen of the inner tube **5** to be in fluid contact with the annular lumen **18** so that liquids, e.g. blood,) can be withdrawn or collected through the catheter **10** from the annular lumen **18.** The port **15** may also act as an entry/exit point for a guidewire.

Whether the second medical device is provided with one or more ports, the size of the ports **15** and the diameter of the inner tube **5** should be selected such as they are not obstructed by the particles if the treatment agent is delivered in said particles. Preferably, the ports are at least 1 mm wide and the diameter of the inner tube is at least 5 mm.

The diameter of the inner tube is preferably > 1mm to prevent the piling up of particles which can occlude the device. The normal blood flow through the liver is about 1.5 - 1.8 liters/minute. The second retrievable medical device have a French size diameter of F18 (= about 6 mm), preferably the diameter is below F16 (= about 5.3 mm), thereby allowing a flow up to 2 liters/minute.

The skilled person will understand that the connection between the inner tube **5** and the ports **15** can be optimized so that expansion of the tubular member **9** does not result in axial tension in the inner tube **5,** or excessive slack along the inner tube **5.** According to one embodiment of the invention, the inner tube **5** of the catheter **10** extends from the outer tubing **3** and is in fluid connection with the annular lumen **18** via one or more ports **15.** In other words, the inner tube **5** may extend from the outer tubing **3** to connect with the ports **15,** as a continuous extension of the inner tube. According to another embodiment of the invention, the inner tube **5** of the catheter **10** is in fluid connection with a port **15** of the tubular member **9** using a bridging tubing.

**FIG 5** shows one configuration of the inner tube **5** whereby a rigid bridging tube **19** is employed to connect fluidly a port **15** of the tubular member **9** to the inner tube **5** of the catheter **10.** **FIG 6** shows an alternative configuration of the inner tube **5** whereby an axially expandable bridging tube **19'** is employed to connect fluidly a port **15** of the tubular member **9** to the inner tube **5** of the catheter **10.** The latter bridging tubing **19'** is typically made from a flexible material which can expand by virtue of elastic properties and/or by use of a concertina-like folding of the unexpanded bridging tube **19'.**

**FIGS. 3A** and **3B** illustrate the system where the tubular member **9** is deployed within a vessel **30.** The proximal **13** and distal **12** annular ridges contact the wall of the vessel **30,** and the central part **11** forms an annular lumen **18.** Blood (illustrated by arrow **b)** is able to flow freely through the unlined portion of the tubular member **9.** The annular lumen **18** may span the region of branched vessels **31** to **37,** wherein blood with therapeutic agent excess (illustrated by arrows **a)** will flow. Blood with therapeutic agent excess (illustrated by arrows **a)** flows then inside the inner tube **5.**

In **FIG. 3A**, the liner **1** is attached to the carrier **2** such that the liner is on the inside of the carrier **2.** **FIG. 3B** shows an alternative configuration whereby the liner **1** is on the outside of the carrier **2.**

The device as described above is particularly useful for the minimal invasive and repeatable treatment of an organ. After positioning, as mentioned, before the device expands to achieve its dumb-bell or hour-glass shape. The proximal annular ridge **13** and distal annular ridge **12** (i.e. the end parts of the device) expand until they press against the inner wall of the vessel, thereby fixing the device at the selected position and providing a liquid tight seal inside the vessel. The central part **11** of the device expands to a lesser degree, thereby creating an annular lumen **18** between the device and the inner wall of the vessel. Inside the device is a passageway lumen **14** for bypassing the systemic blood past the sealed area. This way the passageway lumen **14** defines a new blood path to allow the continuation of the systemic blood flow during perfusion. The liquid tight sealing of the vessel by the proximal annular ridge **13** and distal annular ridge **12** and the liquid tight liner **1** of the device form a liquid tight barrier, separating the systemic blood flowing through the passageway **14** from the blood present in the annular lumen **18.** This blood can be collected from the annular lumen **18.**

Depending on the type and positioning of the cells for treatment, the practitioner can decide in which vein or vessel to introduce the second medical device. During radioembolization of liver, the second medical device id introduced in the vena cava (VC), thereby isolating the blood brought to the VC by the HV.

Although the following describes the use of this system in a retrograde liver perfusion application, the application is only exemplifying. It is clear to a person skilled in the art that this system can also be used for treating other organs than the liver, this orthograde as well as retrograde.

In another preferred embodiment, the second retrievable medical device is used for temporarily isolating a portion of the vasculature from both blood flow and pressure. In the current practice, when a portion of the vasculature needs to be temporarily isolated from both blood flow and pressure in order to repair a damaged vessel or to perform an end-side anastomosis with a synthetic or natural graft, a surgeon needs to place cross-clamps to occlude all blood flow. Occlusion balloons can be placed in the aorta; however, a balloon has a tendency to migrate due to the aortic pressure. For example, when a graft is needed to bypass a chronically occluded iliac artery, a preferred bypass is from the aorta near the bifurcation to the femoral. This is currently performed using an occlusion balloon in the aorta. The problems with using a balloon are that the legs can become ischemic and the balloon has a tendency to migrate due to the aortic pressure. The second medical device can be deployed between in the patient iliac and the aorta and the pressure and blood would be excluded from a portion of the aorta thereby allowing the anastomosis of the bypass graft to be performed without excessive bleeding. The use of the second medical device eliminates both mentioned problems as the blood would be shunted to the legs and there would be no buildup of pressure to cause a device to migrate. Cross-clamps can also be used on the aorta. However, cross-clamps can damage the intimae and if there is calcification in the vessel, it can cause an embolization. The second medical device is less traumatic to the vessel.

**Fig. 16A** shows a vessel **131** wherein a lesion **130** is present. In order to repair the vessel **131,** the second medical device with or without the inner tube 5 is introduced in the vessel (**Fig. 16** **B**) thereby providing the surgeon with a working space to repair the lesion **130.** The impermeability of the liner results in very small or no blood leakage normal blood flow **BF** is not interrupted.

### Third retrievable medical device

The kit of the present invention further comprises at least one more retrievable medical device, called hereafter third retrievable medical device. The third medical device will be used to occlude vessels of the treated organ such as to isolate the organ from the systemic blood flow and can be used as connections for perfusion and/or shunting, e.g. Veno-venous bypass.

When liver is treated for example, the main blood vessels connected to the liver are occluded: the vena porta (PV, hepatic portal vein) using the third retrievable medical device, hepatic artery (HA) using the first retrievable medical device and hepatic vein (HV) using the second retrievable medical device to achieve site specific blood isolation and collection. The isolation of the liver vascular system makes it possible to reach high local chemotherapy concentration.

The introduction of the third retrievable medical device and the first medical device can be achieved by means of an introducer sheath. In a preferred embodiment, the third device incorporates a dilator and eliminates the need for a separate introducer sheath. The third retrievable medical device in the PV has a bypass lumen, e.g. for portal veno-venous bypass, an occlusive seal, preferably a balloon, and a lumen for organ perfusion. The PV medical device may be provided with a perfusion port and associated tubing. The seal is placed upstream of the veins into which the PV branches. Due to its position between the liver and the intestinal parts, the PV is difficult to enter. Therefore, the PV can be entered through the liver as practiced for the placement of a Transjugular Intrahepatic Portosystemic Shunt as is commonly understood by one skilled in the art.

The third retrievable medical device in the PV isolates the abdominal blood flow from the perfusion flow in the liver and is the junction for the Veno-Venous-Bypass for draining off blood coming from the abdominal area. The device also represents the connection point for perfusion from or to the portal vein from the organ side.

In the prior art, two devices are placed in the PV: a balloon catheter that is used to occlude the PV flow and allow aspiration through a central lumen for portal veno-venous bypass, and a drainage catheter for the perfusion circuit. Access to the portal vein should be made through an intercostal puncture site using standard interventional techniques with Duplex-Ultrasound guidance and an appropriate angiographic needle set. If intra-portal pressure measuring is desired and it is anatomically feasible, two access sites should be made. The access site should be dilated and two 12 F or 14 F sheaths are placed in the portal vein. The PVD for bypass is introduced into the first sheath. Veno-Venous-Bypass flow goes through the PVD device and the pressure monitoring is done through the side arm port of the sheath where the PVD is placed. The second 12 F sheath is used for perfusion or can be used as an access sheath for the drainage catheter. Perfusion can occur through the drainage catheter, if desired, to prevent the tip from being sealed against the vessel wall. To position two devices in the portal vein is a procedure that requires experience in interventional technologies, presents increased risk for the patient. Moreover, the punction sites need to be occluded afterwards with appropriate materials.

The third retrievable medical device of the present invention is a single device suitable to isolate and perfuse the PV. Hence, the patient risk is reduced and procedure time is shortened.

The device is shown in **Fig. 13****.** It is a single device, have small diameter, preferably <F12 (about 4 mm) and easy to place under ultrasonic guidance. The device is provided with at least one balloon that occludes the PV. The device also serves as perfusion and/or infusion catheter for the liver perfusate and can additionally facilitate portal veno-venous bypass.

A further detailed illustration of the third retrievable medical device is shown in **Fig. 9** and **Fig. 10****.** The third retrievable medical device comprises a tube having a lumen **58** and an inflation lumen **61.** The diameter **O** of said lumen **58** is at least 2 mm while the diameter **P** of the third retrievable medical device is about 3 mm. The diameter **Q** of proximal end **Y** of the third retrievable medical device is about 4 mm. The lumen **58** is provided at the proximal end **Y** with a female luer adapter **51** and a 4-way hi-flow stopcock **52.** The device is provided with a marker band **60** and a balloon **59** that inflates when the user pushes the inflation bladder **54.** The latter is provided with an inflation check valve **53** and a male luer adapter **55.** The inflation bladder **54** is connected to the inflation lumen **61** via a female luer adapter **56** and a connector tube **57.** In the embodiment shown in **Fig. 9****,** the outlets **62** allowing the perfusion and/or drainage are positioned at the distal end **X** of the balloon **59** of the device and the distance **J** at the distal end **X** and over which the outlets **62** are distributed is comprised between 10 and 14 mm. In the embodiment shown in **Fig. 10****,** the outlets **64** allowing the perfusion and/or drainage are positioned at the proximal end **Y** of the balloon **59.** The distance **T** over which the outlets **62** are distributed is comprised between 15 and 20 mm and the distance **S** at the distal end **X** of the balloon is comprised between 4 and 6 mm. The balloon length L is comprised between 10 and 50 mm, preferably between 15 and 40 mm, more preferably between 20 and 30 mm, most preferably around 24 mm. The Veno-Venous-Bypass of the device is preferably up to 800 cc/min while the normal practice setpoint about 400cc/min, and the perfusion flow up to 400 cc/min while the normal practice setpoints in the range of 100-200 cc/min. In a preferred embodiment, the third medical device is made of a biocompatible materials generally applied for short term, < 60 minutes, endovascular procedures.

### Separation device

The kit of the present invention comprises a separation device for separating the therapeutic agent excess from the collected blood thereby removing the excess of therapeutic agent. The separation device is provided with an inlet and an outlet and at least one filter. The filter is able to mechanically filter particle-contamination, chemically bond chemo-contamination or a combination of both. The inlet of the separation device is in fluid communication with the proximal end of the inner tube of the second medical device. Consequently, the blood containing therapeutic agent excess is directed through the inner tube to the separation device wherein filtration takes place. The filtered blood flows out of the device through the outlet of the separation device. The filtered blood joins the systemic blood flow or is redirected to the systemic blood flow if the separation device is extracorporeal. In a preferred embodiment, the separation device comprises at least one filter provided with openings which size is at least 15.

In another preferred embodiment, the separation device comprises at least two filters wherein the first filter, through which the collected blood flows, is provided with opening larger than the openings of the second filter through which the collected blood flows. The openings of the first filter are at least 15% larger than the openings of the second filter through which the blood will flow.

In an embodiment not forming part of the invention such as claimed, the separation device is positioned in the second medical device as presented in **Fig. 3A** and **Fig. 3B****.** The separation device **40,** in this case will consist of at least one filter provided with openings which size is comprised between 15 and 65 micrometers, preferably between 20 and 30 micrometers, more preferably around 25 micrometers. The filter is connected to the inner tube **5** proximal end of the second retrievable medical device. In this embodiment, the filter can be made of a flexible material suitable to be deployed when the second medical device is to be retrieved from the patient's vessel **30** at the end of the treatment. The separation device **40** can be provided with an internal container for storage of the therapeutic agent that is separated from the blood. This is preferably used when the therapeutic agent is contained in particles which are delivered to the organ.

In an embodiment according to the invention such as claimed, the separation device is positioned in the second medical device as presented in **Fig. 6** and **Fig. 7****.** The separation device, in this case will consist of at least one filter **37** provided with openings which size is comprised between 15 and 65 micrometers, preferably between 20 and 30 micrometers, more preferably around 25 micrometers. The filter **37** can be made of a flexible material suitable to be deployed when the second medical device is to be retrieved from the patient's vessel at the end of the treatment. In this embodiment, the hepatic blood, with free therapeutic agent **50,** is leaving the hepatic veins, through the annular lumen **18,** and is filling an area **36** surrounded by a filter material **37 (****Fig. 6****).** Particles or other therapeutic agents will be trapped by the filter. The hepatic venous blood or the hepatic outflow will pass through the filter and will join the vena cava blood flow. The area surrounded by filter material **37** could be connected to the inner tube **5** of the catheter. During filtering, the inner tube **5** will be closed. The venturi effect created due to the narrowing causes an underpressure at the catheter side; this is actively supporting the filter capacity. When the filtration is to be terminated and the second medical device to be withdrawn from the vessel, the inner tube **5** of the catheter is shortly open to drain the captured therapeutic agents out before retrieving the device. This could be done also a few times during the procedure when there is a risk for overload or when a fast exit is needed.

In another embodiment shown in **Fig. 7****,** the filter **37** is positioned at the inner proximal end of the tubular member **9.** The filter **37** is covering at least the inner proximal end that is devoid of liner **1.** The device is prepared for retrieving from the vessel by pushing the outer tube **3** over the tubular member **9.** This will reduce the diameter of the tubular member **9;** the narrow side of the tubular member **9** will close off the filter **37** assuring that captured agent remains in the filter area **41.** When tubular member **9** is completely loaded into the outer tube **3,** it can be retrieved with the filtered agent captured inside.

In another preferred embodiment, the separation device is located in the second retrievable medical device. In this embodiment the therapeutic agent is delivered using particles or beads. In a further preferred embodiment, the cavity also called annular lumen **18** created by the second medical device around the outlet of the hepatic vein is partly or completely constructed of a filter material. Said filter material is able to mechanically filter particle-contamination, chemically bond chemo-contamination or a combination of both. The captured material will be made non-harmful at the site where it is collected or will be retrieved when the second medical device is retrieved. The filter can be attached to the region of the cavity **18** formed by the second retrievable medical device. In this way the blood containing particles excess flowing from the organ outflow or from the veins **31-37 (****FIG. 3A** and **3B**) will first be filtered due to the presence of the filter then will flow in through the fluid ports into the inner tube **5** of the second retrievable medical device. In this embodiment, the inlet of the separation device is the side of the filter that comes in contact with the particles excess blood while the outlet of the separation device is the side of the filter from which filtered blood flows. The filter is provided with openings which size is comprised between 15 and 65 micrometers, preferably between 20 and 30 micrometers, more preferably around 25 micrometers. A pump can be provided to move the blood at relatively constant low pressure, the object being to avoid raising or lowering the fluid or blood pressure.

In an embodiment not forming part of the invention such as claimed, the separation device is provided with the necessary connection so as to form at least a partially extracorporeal filtering circuit. This embodiment is illustrated in **FIG. 4****;** the proximal end **20** of the second retrievable medical device is connected to the separation device **25** inlet **38.** A pump, such as a roller pump or centrifugal pump, might be provided to establish a negative relative pressure in the liver **27,** thereby collecting the contaminated blood through the inner tube **5** of the second retrievable medical device. The pump can be a pump such as e.g. a roller pump or centrifugal pump, but frequently a heart lung machine is used. The collected blood is then directed to the separation device **25** wherein it will be separated from the therapeutic agent thanks to the presence of the filter.

The pump moves the blood through an extracorporeal circuit at relatively constant low pressure, the object being to avoid raising or lowering the fluid pressure of the total circuit ranging from the vena cava through the return to the body. The venous blood or the outflow blood is transported through tubing, optionally into the separation device **25.** The filtered blood leaves the separation device **25** through its outlet **39** and is transported through tube **28** and returned to the patient at the level of the right atrium (RA) by standard procedures in the art (**FIG. 4**).

The separation device **25** could be an already commercially available device like the "Affinity Arterial Filter", supplied by Medtronic.

Furthermore, temperature regulators, oxygenators and/or other equipment are preferably connected to the filtering circuit. In this circuit the contaminated blood is pumped from the vena cava into the separation device **25.** After filtration, the filtered blood is directed back to the patient's blood flow through the space between the inner tube **5** and the outer tube **3** of the second retrievable medical device. The filtered blood is redirected to the outer tubing **3** of the second retrievable medical device which delivers the filtered blood in the blood flow of the right atrium.

In a preferred embodiment, at least one pump is suitable to be connected to the proximal end of the second retrievable medical device catheter. In a further preferred embodiment, at least one blood oxygenator is suitable to be connected to the separation device **25** outlet **39.**

The extra-corporeal filtering circuit comprises at least two tubes for providing connections which will direct the contaminated blood from the inner tube **5** of the second medical device to the separation device **25** and for redirecting the filtered blood from the separation device **25** into the patient blood flow. These connections can be luer lock or screw connections, but also any other types of connections suitable to be used and known to the person skilled in the art are conceivable. The connections of the extra-corporeal filtering circuit assure sterility in a potentially unsterile environment and a resistance to mechanical stresses for the duration of the treatment with conscious patients.

The filtration of blood can take place using a combination of an inner separation device, positioned in the second retrievable medical device according to any of the above described embodiments, and an external filtration as shown in **Fig. 4****.**

In a second aspect, the present invention provides a method for the delivery a therapeutic agent to an organ blood flow and the removal of the excess of said therapeutic agent from said organ blood flow. The method comprises the steps of (a) introducing a first medical device **(26,** **FIG. 4****)** in the organ artery or organ inflow vessel, (b) introducing a second medical device in the organ vein or organ outflow vessel, without obstruction of the systemic blood flow, (c) controlling the infusion and/or the perfusion flow, thereby optimizing the settings for maximal local therapeutic effects, with minimal collateral damage, (d) injecting the therapeutic agent into the organ artery or organ inflow using the first medical device, (e) collecting the organ vein blood or the organ outflow having a therapeutic agent excess using the second medical device, (f) circulating the drug loaded perfusate, thereby optimizing the therapeutic results by adjusting the physical parameters and adding or removing therapeutic additives, and (g) separating the therapeutic agent excess from the collected organ outflow using the separation device. The method further comprises the step of (h) redirecting the filtered blood into the organ blood flow if an extercorporeal blood filtration is performed.

In a preferred embodiment, the present invention provides a method for perfusion of a therapeutic agent to an isolated organ wherein the perfusion parameters, such as flows, pressures, durations etc, are automatically adjusted. According to the current invention, there is no need for a team comprising at a minimum a perfusionist, anesthesiologist and nuclear radiologists to monitor and control the perfusion. The method of the invention reduces the number of people required and simplifies a very complicated medical procedure by using an algorithm to adjust the used machines and to minimize the leak rate of the perfused therapeutic agent to the systemic circulation and automate the various processes of the perfusion.

The method provided by the invention is novel as there are no real time leak test methods and leak control systems available today. Nowadays, to secure a minimum safety level, it is needed to inject the patient with a radioactive load, a technical specialist will continuously measure the radioactive load in the perfused organ and the systemic blood flow, evaluate the possibility of drug and isotope loaded perfusate leaking to the patients system. The technical specialist verbally warns the medical doctor in charge. The latter assigns the perfusionist to adjust the settings to get a control over the situation again or decides to start immediately a washout of the perfusate ending the procedure. The method of the invention provides a fast and adequate corrective action which ensures patient's safety.

In addition to «leak » correction », the method of the invention provides a continuous measurement of the liver parameters which are used as input and control of flow-pressure, drug regimes, additives etc. The method thus provides an optimized treatment wherein real time measurements are used for an immediate reaction from the practitioner.

Up to date, the local perfusions need a large group of clinical specialist to execute a full procedure: interventional radiologist, anesthesiologist, nuclear specialist, perfusionist, oncologist, nurses. Moreover, due to the full manual control, the procedure could take more than 4 hours to be completed. The local perfusions have to be performed 2 to 5 times per patient. All these factors result in a treatment which is expensive. Therefore, the treatment cannot be made available due to all patients for economic reasons. The method provided by the invention overcomes the described problem as it provides a more patient safe and cheaper treatment.

In a preferred embodiment, once the organ has been isolated from the systemic circulation, the method uses a system where the intra-organ blood pressure and leak rate to the systemic circulation are monitored and used in an algorithm to control the perfusion dynamics of a therapeutic agent perfusion i.e. pump speed and/or pressures, flow balancing and metering and, potentially, drug administration.

In the method provided by the invention, the blood flows into and out of the organ that is isolated. The method comprises the step of determining all major blood sources into and out of the organ. Before the procedure the perfusion path must be determined. The perfusion_can take place orthograde or retrograde. Some vessels may be only occluded and not used, if desired. The perfusion path is dependent upon the organ being treated and will have to be determined prior to clinical use. The method can also be applied to open or partially open procedures especially when it is not possible to access an organ percutaneously. For percutaneous access, a vessel is located using ultrasound and accessed with a guidewire using the Seldinger technique. Once a vessel has been accessed an introducer sheath can be placed. The normal blood flow can then be controlled using occlusion balloons with central lumens for the perfusion or stent-like devices to isolate the blood flow to or from the organ from the systemic blood flow. If the blood flow is occluded, it may be desirable to shunt the blood from the systemic side back into the systemic circulation to minimize the possibility of thrombus formation. The effectiveness isolation of the vessel should be verified angiographically when possible. Once organ isolation has been verified angiographically, the perfusion can start.

The method provided by the invention comprises the step of connecting the perfusion devices to the perfusion system and the control algorithm can be started. The perfusion system is primed with either blood or physiologic solution as needed, in order to prevent any air from entering the perfusion circuit. The perfusion system consists of at least one roller or centrifugal pump(s) that pull blood from the organ to be treated. The blood is collected into a storage reservoir. Perfusate is pumped out of the reservoir and back into the organ. Optionally, the perfusate can be pumped through a heat exchanger, to make it warmer or colder as desired and/or an oxygenator prior to being returned to the organ.

In the method provided by the invention, the intra-organ pressure can be measured through any static lumen that is in direct contact with the perfusion blood circuit. The pressure is input into the control algorithm and the pumps to and from the organ are adjusted so that there is a negative balance, i.e. the flow from the organ is higher than the flow to the organ. It has been found through in-vivo testing in an animal model that if the intra-organ pressure can be reduced below the systemic pressure, the leakage from the perfusion circuit to the systemic circuit can be reduced or eliminated. The control algorithm will adjust the flows until the desired intra-organ pressure is obtained. Once the desired intra-organ pressure is obtained, the leakage monitoring can start.

Isolation of the organ in the method provided by the invention can be continuously monitored several ways. First a radiomarker, such as ^{99m}Tc) is bound to a larger molecule, such as albumin or red blood cells, in order to prevent migration through cell walls that may indicate a false leak. The radiomarker can be detected using a NaI crystal scintillation counter or similar. One method of detection uses a single NaI detector placed over an area of the body that has high blood flow but is sufficiently far from the organ being perfused to prevent background noise, e.g. the groin area if treating the liver. A small amount of the radioisotope is first injected into the systemic circulation for calibration purposes. A larger amount of radioisotope, e.g. 10x the calibration dose, is then injected into the isolated organ. The NaI detector is continuously monitored by the control algorithm and any leakage to the systemic circulation is minimized by adjusting the perfusion dynamics.

Another method of nuclear detection uses two NaI scintillation counters placed in-line. The detectors are housed in lead shielding to prevent the possibility of any background noise from the perfused organ. One detector is placed in line on the perfusion circuit and a second detector is placed in-line on any extracorporeal systemic shunts, e.g. a veno-venous bypass, if present. A small amount of radioisotope is injected into the systemic circulation prior to use for calibration purposes. The leakage to the systemic circulation can be detected by the detector on the systemic shunt and verified by the detector on the perfusion equipment. The advantage of this system is that there is essentially no possibility of background measurement in the systemic circulation from the organ being perfused. Additionally, the second detector on the perfusion circuit will verify the presence of a leakage to the system, the counts will decrease on the perfusion detector and the inverse will happen on the systemic detector. A variation of this setup is if there is not a systemic shunt made. In this case, the systemic detector can be placed over an area of the body that has high flow as in the first method described. The output from the detectors is monitored by the control algorithm and any leakage to the systemic circulation is minimized by adjusting the perfusion dynamics.

In the method provided by the invention, once the leak rate to the systemic circulation has stabilized at an acceptably low value, the therapeutic drug is administered into the perfusion circuit. This can also be performed automatically as part of the control algorithm. If possible, the uptake of the therapeutic agent can be monitored by the control algorithm and the perfusion parameters adjusted to obtain the correct pharmacokinetic response.

The method provided by invention then allows for and monitors the perfusion for a specific time period. At the end of the time period the control algorithm can start a wash out procedure to remove any therapeutic drug in the perfusate. This can be done by prescribing a time and/or volume of clean blood or physiologic solution to replace the perfusate or by another method, depending on the application.

After the therapeutic agent has been removed from the perfusate, the perfusion equipment is disconnected from the isolation catheters/devices. The devices are then retrieved and normal blood flow is restored to the patient. All access sites are closed and the procedure is completed.

**Fig. 14** illustrates a method for perfusing an organ **101** using a system of electro-mechanical actuators and sensors that are controlled by an algorithm. Once the organ has been isolated using percutaneous interventional techniques, the isolation catheters are attached to the outflow tubing **102** and inflow tubing **103** of the perfusion system. Prior to connecting the devices to the perfusion system, the isolation of the organ has been verified angiographically. The perfusion is primed with blood or physiologic solution as desired prior to use.

Blood is pumped from the organ using pumps **104** and **105.** The number and type of pumps required is dependent upon the organ being treated. The pumps can be either roller pumps which are flow controlled or centrifugal pumps which are pressure controlled. The purpose of pumps **104** and **105** are to pull blood from the organ, or create suction from the organ. It is also possible for a single pump to pull blood from several different vessels, as desired by the clinician. The blood that is pulled from the organ can then be run through a blood filter reservoir **106** if desired. This reservoir is used to remove any bubbles or micro emboli that may be present in the blood, and it serves as a buffer of perfusate. The filtered blood is then collected in reservoir **107.** Blood from the reservoir is pumped **108** into an optional heat exchanger **109** and into an optional oxygenator **110.** The need for hypo or hyperthermia and/or oxygenated blood is based on the requirements of the medical treatment and application and may not always be necessary. Similar to the outlet pumps, more than one pump **108** can be used to return blood to the organ if desired. This pump is preferably a roller pump, but a centrifugal pump may be better for some applications.

From the oxygenator, the blood then flows through injection manifold **111** and bubble trap **112.** The injection manifold can be connected to syringe pumps as needed to administer radiomarker and medical treatment e.g. chemotherapy or heparin, as needed. The syringe pumps connected to manifold **111** can be controlled by the algorithm to administer medical treatment at predefined points or as a result of continuous blood monitoring. From here the blood is returned to the organ.

The method provided by the invention also allows for various sensors, valves, metering pumps, or flow meters **113, 114, 115, 116** to be placed on the outflow and in flow to monitor and control the flow as desired by the algorithm. The level of reservoir **107** is also monitored by the algorithm and can be added to **117** if the level gets too low. There is an overflow **118** on the reservoir that can collect excess perfusate, if necessary, for example during the washout step.

Nuclear detectors **119** and **120** can be placed on the perfusion circuit and the systemic circulation. Ideally both detectors are housed in lead shielding with the perfusion line to avoid any background interference. This will only be possible in the systemic circulation if there is an extracorporeal shunt made e.g. a veno-venous bypass. If there is no shunt, detector **120** is located over an area of the body with high blood flow that is sufficiently far away from the organ being treated to prevent interference. The control algorithm can control the administration of radioisotope and perform all the leak rate calculations.

The control unit **112** is where the control algorithm is programmed and will have a graphical interface for the user. In order to control the method of the invention, the user will be required to input critical information. Such information may be: target perfusion flow rate, acceptable flow range, intra-organ pressure, maximum leak rate, and perfusion time. The control algorithm can use feedback loops to control the pump speeds to obtain the desired intra-organ pressures and leak rates. Additionally, the control unit will automate the steps of the perfusion as follows: first the flow rate into and out of the organ will be increased until the target flow rate is obtained. During this procedure, the flow meters **113, 114, 115** and **116** can be read to assure the tubing has not collapsed or the flow has not been compromised. During in-vivo animal testing, monitoring the perfusion tubing for adequate flow consumed a lot of time. If there is a problem with the flow, the user will be notified so that the problem may be corrected. Once the flow is acceptable the control algorithm will begin the process of lowering the intra-organ pressure **123** to the target value. This is done by adjusting the negative balance of the perfusion pumps. This can be done using a feedback controller e.g. a PID controller. During this time the patency of all perfusion lumens is verified by flow meters. The control algorithm can then start the leakage monitoring by administering the radioisotopes and controlling the leak rate as was previously described. At the end of the perfusion period, the control algorithm can actuate valve **121** and rinse the organ with clean blood and/or physiologic solution **122.** The method of the invention also allows for the connection of other sensors, actuators or pumps as needed **124.** Such sensors might be used for conducting in-process assays to determine drug concentrations or for blood analysis as needed.

Organs can be isolated and perfused using the first, second and third medical devices as described above. To control a local, isolated, perfusion or infusion procedure it is however necessary to deal with patient specific vasculature, collateral connections, to control the communication between the perfusion or infusion liquid and the patients system.

**Fig. 15** is a flowchart of an algorithm that can be used to control the perfusion system of liver using a method of the present invention. The flowchart describes the different steps of the method that have to be performed after isolating the liver using the first, second and third medical devices as described above. N and Y in the figure respectively refer to "No" and "Yes".

The method provided by the present invention provides an auto-controlled optimal perfusion flow wherein a local treatment is optimized in relation to organ isolation and a controlled auto stop when the measured or calculated leak is higher than a preset value. This preset value could be for instance a % of the maximum systemic allowed dose. The total leakage should be limited assuring that with the leakage, the maximum allowed systemic dose remains below the defined maximum values, preferable <40% of the systemic allowed maximum, more preferable < 10%.

The method provided by the present invention is programmable and comprises the following steps:
a. detecting real Time Leak using isotopes, chemicals, photons, light, acoustics, contrast agents, etc,
b. running different cycles with different parameters such as temperature, drugs, additives, time, etc,
c. Washing out step.

In a third aspect, the present invention provides for the use of the kit as described above and the method as described above for the delivery a therapeutic agent to an organ blood flow and for the removal of the excess of said therapeutic agent from said organ blood flow.

The present invention allows minimal invasive delivery of therapeutic agent to an organ of a living, such as liver, and removal of the excess of said therapeutic agent. The removal is assured by positioning the second retrievable medical device into the inferior vena cava, thereby: (a) blocking the flow path of blood in the inferior vena cava; (b) providing a flow path for the blood through the passageway **14** of the device; (c) creating an isolated cavity (annular lumen) **18** between the medical device and the wall of the vena cava, which is in contact with the liver by the hepatic veins; and (d) separating the therapeutic agent from the blood flowing in this cavity by means of the separation device.

The kit of the present invention and the related devices and method allow the closing of systemic and liver originated collateral connections between renal veins (RV) and the right atrium to prevent uncontrolled discharge of beads containing the therapeutic agents. The present invention also assures that the systemic blood flow through the vena cava, lower extremities up to the right atrium, is not interrupted. The second medical device of the present invention is developed to be positioned in front of the hepatic veins percutanously, and retrieved again, this is performed using minimal invasive techniques to allow repetitive procedures in a relatively short time.

The present invention further assures a secure close-off of the blood outflow from the hepatic veins into the vena cava to prevent the therapeutic agent and or particles etc. that had been injected into the liver and are to be eliminated, to get into the right atrium. Whereby occlusive balloons have shown that they are leaking towards the right atrium or are pulled into the right atrium because of the short sealing distance between diaphragm and right atrium, what can provoke severe side effects like e.g. extra-systoles or leakage. The sealing distance of the second medical device is located at the proximal end of the liner and is comprised between 5 and 50 mm, preferably between 8 and 45 mm, more preferably between 15 and 35 mm, most preferably around 21 mm.

The kit of the present invention and the related devices and method ensure the occlusion of collateral vessel connections draining into the Vena Cava between the renal veins and the right atrium. Meanwhile, blood flow through the vena cava is maintained without causing hypovolemia. The maintained flow through the vena cava is minimally 70% of the normal VC flow. An adequate isolation between contaminated hepatic blood and vena cava blood flow is assured using the kit, devices and the method of the present invention.

Although the present invention has been described with reference to preferred embodiments thereof, many modifications and alternations may be made by a person having ordinary skill in the art without departing from the scope of this invention which is defined by the appended claims.

## Claims

1. A kit for the delivery of a therapeutic agent to an organ blood flow and the removal of the
excess of said therapeutic agent from said organ blood flow, said kit comprises:
a. optionally a therapeutic agent which is a fluid treatment and/or particles comprising a radioactive element, the size of said particles is comprised between 20 and 60 micrometers,
b. a first retrievable medical device (26), for delivering said therapeutic agent into the organ inflow blood, comprising a catheter and an injection device, said catheter having a proximal end, a distal end and a lumen; the distal end is in fluid communication with the proximal end via the lumen; said injection device is suitable to be connected to the proximal end of the catheter,
c. a second retrievable medical device for isolating the organ outflow, having a distal end (21) and a proximal end (20); said second medical device comprises a catheter (10) suitable for deploying a self-expanding hollow tubular member (9); the proximal end of the tubular member (9) is attached to the distal end of the catheter (10); said tubular member (9) is configured to expand radially to form a central part (11) flanked by two annular ridges, a distal annular ridge (12) and a proximal annular ridge (13) wherein the central part (11) radially expands to a lesser degree compared to the annular ridges (12, 13); said tubular member (9) comprises a liquid-impermeable area disposed with one or more fluid ports (15) for the collection of the organ outflow and two liquid-permeable regions, one distal to the distal annular ridge (12) and one proximal to the proximal annular ridge (13) so forming a passageway (14) between the distal end and the proximal end of the tubular member (9) for the flow the organ blood devoid of therapeutic agent, whereby the liquid-impermeable area is disposed in an area defined by the region flanked by the annular ridges (12, 13), the distal annular ridge (12) and at least part of the proximal annular ridge (13) isolating the organ outflow containing therapeutic agent excess from the organ blood flow devoid of therapeutic agent, and
d. a separation device (25) comprising at least one filter able to separate the therapeutic agent excess from the organ outflow, having an inlet (38) for the organ outflow having a therapeutic agent excess and an outlet (39) for filtered organ outflow blood,
**characterised in that** the separation device (25) is located in the second retrievable medical device, said separation device comprising at least one filter which is positioned at the inner proximal end of the tubular member (9) of the second retrievable medical device.

2. The kit according to claim 1, wherein the tubular member (9) of the second medical device comprises a liquid permeable carrier (2) configured to adopt an essentially cylindrical state when compressed and to self-expand radially to form said tubular member (9).

3. The kit according to any of claims 1-2, wherein the tubular member (9) of the second medical device comprises a liner (1) attached to at least a part of the wall of the tubular member, which provides the liquid impermeable area.

4. The kit according to any of claims 1-3, wherein the catheter (10) of the second medical device comprises an inner tube (5), an outer tube (3) surrounding at least a portion of the length of the inner tube (5) and a pusher means (23) for the deployment of the tubular member (9); the distal end of the pusher means (23) is attached to the proximal end of the tubular member (9).

5. The kit according to claim 4, wherein the inner tube (5) distal end of the catheter (10) of the second medical device is in fluid connection with the liquid-impermeable area of the tubular member (9) and the inner tube (5) proximal end is in fluid connection with the separation device inlet.

6. The kit according to claim 2, wherein the carrier of the second medical device is made of a braided wire mesh.

7. The kit according to any of claims 1-6, wherein the separation device (25) comprises at least one filter provided with openings which size is comprised between 15 and 65 micrometers, preferably between 20 and 30 micrometers, more preferably around 25 micrometers.

8. The kit according to any of claims 1-7, wherein the separation device (25) comprises at least two filters, a first filter and a second filter.

9. The kit according to any of claims 1-8, wherein the openings of the first filter are at least 15% larger than the openings of the second filter through which the blood will flow.

10. The kit according to any of claims 1-9, comprising at least one pump suitable to be connected to the proximal end of the second retrievable medical device catheter (10).

11. The kit according to any of claims 1-10, comprising at least one third retrievable medical device for the isolation and/or the perfusion of veins, said third retrievable medical device comprises a lumen (58), an inflation lumen (61), a balloon (59) at the distal end (X) of the device and a plurality of outlets (62), said outlets (62) are positioned at the distal end (X) of the balloon (59) or at the proximal end (Y) of the balloon (59).

12. The kit according to any of claims 1-11, wherein the first retrievable medical device comprises at least one inflatable balloon (65), said balloon (65) is inflated by flowing therapeutic agent in the lumen of the device and thorough openings (68, 69) provided in said device.

## Patentansprüche

1. Ein Kit für die Abgabe eines therapeutischen Mittels an einen Organblutfluss und die Entfernung des Überschusses des therapeutischen Mittels von dem Organblutfluss, wobei das Kit Folgendes umfasst:
a. gegebenenfalls ein therapeutisches Mittel, das als Behandlungsfluid und/oder Partikel vorliegt, die ein radioaktives Element umfassen, wobei die Größe der Partikel zwischen 20 und 60 Mikrometer beträgt;
b. eine erste rückholbare medizinische Vorrichtung (26), zum Abgeben des therapeutischen Mittels an das Zuströmblut des Organs, die einen Katheter und eine Injektionsvorrichtung umfasst, wobei der Katheter ein proximales Ende, ein distales Ende und ein Lumen aufweist; das distale Ende über das Lumen in Fluidkommunikation steht mit dem proximalen Ende und die Injektionsvorrichtung geeignet ist, an dem proximalen Ende des Katheters angeschlossen zu werden;
c. eine zweite rückholbare medizinische Vorrichtung zum Isolieren des Organabstroms, die ein distales Ende (21) und ein proximales Ende (20) aufweist; wobei die zweite medizinische Vorrichtung einen Katheter (10) umfasst, der zum Entfalten eines selbstexpandierenden hohlen röhrenförmigen Elements (9) geeignet ist; und das proximale Ende des röhrenförmigen Elements (9) an dem distalen Ende des Katheters (10) angebracht ist; wobei das röhrenförmige Element (9) konfiguriert ist, um radial zu expandieren und einen mittigen Teil (11) zu formen, der von zwei ringförmigen Rippen, einer distalen ringförmigen Rippe (12) und einer proximalen ringförmigen Rippe (13) flankiert ist, wobei der mittige Teil (11) im Vergleich zu den ringförmigen Rippen (12, 13) in einem geringeren Grad radial expandiert; wobei das röhrenförmige Element (9) einen flüssigkeitsundurchlässigen Bereich, der mit einem oder mehreren Fluiddurchlässen (15) für das Auffangen des Organabstroms angeordnet ist, und zwei flüssigkeitsdurchlässige Zonen umfasst, eine distal zu der distalen ringförmigen Rippe (12) und eine proximal zu der proximalen ringförmigen Rippe (13), um so einen Durchgang (14) zwischen dem distalen Ende und dem proximalen Ende des röhrenförmigen Elements (9) für den Fluss des Organbluts, das frei von dem therapeutischen Mittel ist, zu formen, wobei der flüssigkeitsundurchlässige Bereich in einem Bereich angeordnet ist, der durch die Zone definiert wird, die durch die ringförmigen Rippen (12, 13) flankiert wird, wobei die distale ringförmige Rippe (12) und mindestens ein Teil der proximalen ringförmigen Rippe (13) den Organabstrom, der den Überschuss des therapeutischen Mittels enthält, von dem Organblutfluss isolieren, der frei von dem therapeutischen Mittel ist; und
d. eine Trennvorrichtung (25), die mindestens einen Filter umfasst, der den Überschuss des therapeutischen Mittels von dem Organabstrom trennen kann, mit einem Einlass (38) für den Organabstrom, der einen Überschuss des therapeutischen Mittels aufweist, und einem Auslass (39) für das gefilterte Blut des Organabstroms;
**dadurch gekennzeichnet, dass**
die Trennvorrichtung (25) sich in der zweiten rückholbaren medizinischen Vorrichtung befindet, wobei die Trennvorrichtung mindestens einen Filter umfasst, der am inneren proximalen Ende des röhrenförmigen Elements (9) der zweiten rückholbaren medizinischen Vorrichtung angeordnet ist.

2. Das Kit nach Anspruch 1, wobei das röhrenförmige Element (9) der zweiten medizinischen Vorrichtung einen flüssigkeitsdurchlässigen Träger (2) umfasst, der konfiguriert ist, um, wenn zusammengedrückt, einen im Wesentlichen zylindrischen Zustand anzunehmen und selbst radial zu expandieren und das röhrenförmige Element (9) zu formen.

3. Das Kit nach einem der Ansprüche 1-2, wobei das röhrenförmige Element (9) der zweiten medizinischen Vorrichtung eine Auskleidung (1) umfasst, die an mindestens einem Teil der Wand des röhrenförmigen Elements angebracht ist, und den flüssigkeitsundurchlässigen Bereich bereitstellt.

4. Das Kit nach einem der Ansprüche 1-3, wobei der Katheter (10) der zweiten medizinischen Vorrichtung eine Innenröhre (5), eine Außenröhre (3), die mindestens einen Teil der Länge der Innenröhre (5) umgibt, und ein Schiebemittel (23) für die Entfaltung des röhrenförmigen Elements (9) umfasst; wobei das distale Ende des Schiebemittels (23) an dem proximalen Ende des röhrenförmigen Elements (9) angebracht ist.

5. Das Kit nach Anspruch 4, wobei das distale Ende der Innenröhre (5) des Katheters (10) der zweiten medizinischen Vorrichtung in Fluidverbindung mit dem flüssigkeitsundurchlässigen Bereich des röhrenförmigen Elements (9) steht und das proximale Ende der Innenröhre (5) in Fluidverbindung mit dem Trennvorrichtungseinlass steht.

6. Das Kit nach Anspruch 2, wobei der Träger der zweiten medizinischen Vorrichtung aus einem geflochtenen Drahtnetz hergestellt ist.

7. Das Kit nach einem der Ansprüche 1-6, wobei die Trennvorrichtung (25) mindestens einen Filter umfasst, der mit Öffnungen versehen ist, deren Größe zwischen 15 und 65 Mikrometer, bevorzugt zwischen 20 und 30 Mikrometer, besonders bevorzugt ungefähr 25 Mikrometer beträgt.

8. Das Kit nach einem der Ansprüche 1-7, wobei die Trennvorrichtung (25) mindestens zwei Filter, einen ersten Filter und einen zweiten Filter, umfasst.

9. Das Kit nach einem der Ansprüche 1-8, wobei die Öffnungen des ersten Filters mindestens 15 % größer als die Öffnungen des zweiten Filters sind, durch den das Blut fließen wird.

10. Das Kit nach einem der Ansprüche 1-9, das mindestens eine Pumpe umfasst, die geeignet ist, an dem proximalen Ende des zweiten rückholbaren medizinischen Vorrichtungskatheters (10) angeschlossen zu werden.

11. Das Kit nach einem der Ansprüche 1-10, das mindestens eine dritte rückholbare medizinische Vorrichtung für die Isolation und/oder die Perfusion von Venen umfasst, wobei die dritte rückholbare medizinische Vorrichtung ein Lumen (58), ein Inflationslumen (61), einen Ballon (59) am distalen Ende (X) der Vorrichtung und eine Vielzahl von Auslässen (62) umfasst, wobei die Auslässe (62) am distalen Ende (X) des Ballons (59) oder am proximalen Ende (Y) des Ballons (59) angeordnet sind.

12. Das Kit nach einem der Ansprüche 1-11, wobei die erste rückholbare medizinische Vorrichtung mindestens einen aufblasbaren Ballon (65) umfasst, wobei der Ballon (65) dadurch aufgeblasen wird, dass das therapeutische Mittel in das Lumen der Vorrichtung und durch die Öffnungen (68, 69), die in der Vorrichtung bereitgestellt werden, strömt.

## Revendications

1. Un kit pour la délivrance d'un agent thérapeutique à un flux sanguin dans l'organe et l'élimination de l'excès dudit agent thérapeutique dudit flux sanguin dans l'organe, ledit kit comprenant:
a. de manière facultative un agent thérapeutique qui est un traitement à fluide et/ou des particules comprenant un élément radioactif, la taille desdites particules est comprise entre 20 et 60 micromètres,
b. un premier dispositif médical récupérable (26), pour la délivrance dudit agent thérapeutique dans l'afflux sanguin de l'organe, comprenant un cathéter et un dispositif d'injection, ledit cathéter ayant une extrémité proximale, une extrémité distale et une lumière; l'extrémité distale est en communication à fluide avec l'extrémité proximale par la lumière; ledit dispositif d'injection est approprié à être connecté à l'extrémité proximale du cathéter,
c. un second dispositif médical récupérable pour isoler l'écoulement d'organe, ayant une extrémité distale (21) et une extrémité proximale (20); ledit second dispositif médical comprend un cathéter (10) approprié pour déployer un élément tubulaire creux auto-expansible (9); l'extrémité proximale de l'élément tubulaire (9) est reliée à l'extrémité distale du cathéter (10); ledit élément tubulaire (9) est configuré pour s'étendre radialement afin de former une partie centrale (11) flanquée par deux arêtes annulaires, une arête annulaire distale (12) et une arête annulaire proximale (13) dans lequel la partie centrale (11) s'étend radialement à un degré moindre comparé aux arêtes annulaires (12, 13); ledit élément tubulaire (9) comprend une zone imperméable aux liquides munie d'un ou plusieurs orifices de fluide (15) pour la collection de l'écoulement d'organe et deux régions perméable aux liquides, une région distale à l'arête annulaire distale (12) et une région proximale à l'arête annulaire proximale (13), formant ainsi un passage (14) entre l'extrémité distale et l'extrémité proximale de l'élément tubulaire (9) pour le flux sanguin dans l'organe dépourvu d'agent thérapeutique, dans lequel la zone imperméable aux liquides est disposée dans une zone définie par la région flanquée par les arêtes annulaires (12, 13), l'arête annulaire distale (12) et au moins une partie de l'arête annulaire proximale (13) isolant l'écoulement de l'organe contenant de l'excès d'agent thérapeutique du flux sanguin dans l'organe dépourvu d'agent thérapeutique, et
d. un dispositif de séparation (25) comprenant au moins un filtre capable de séparer l'excès d'agent thérapeutique de l'écoulement de l'organe, ayant une entrée (38) pour l'écoulement de l'organe ayant un excès d'agent thérapeutique et une sortie (39) pour le sang filtre de l'écoulement de l'organe,
**caractérisé en ce que**
le dispositif de séparation (25) est situé dans le second dispositif médical récupérable, ledit dispositif de séparation comprenant au moins un filtre qui est positionné à l'extrémité proximale interne de l'élément tubulaire (9) du second dispositif médical récupérable.

2. Le kit selon la revendication 1, dans lequel l'élément tubulaire (9) du second dispositif médical récupérable comprend un transporteur perméable aux liquides (2) configuré pour adopter un état essentiellement cylindrique lors compression et pour s'étendre de lui-même radialement afin de former ledit élément tubulaire (9).

3. Le kit selon l'une quelconque des revendications 1-2, dans lequel l'élément tubulaire (9) du second dispositif médical comprend une doublure (1) reliée à au moins une partie de la paroi de l'élément tubulaire, qui fournit la zone imperméable aux liquides.

4. Le kit selon l'une quelconque des revendications 1-3, dans lequel le cathéter (10) du second dispositif médical comprend un tube interne (5), un tube externe (3) entourant au moins une partie de la longueur du tube interne (5) et un moyen pousseur (23) pour déployer l'élément tubulaire (9); l'extrémité distale du moyen pousseur (23) est reliée à l'extrémité proximale de l'élément tubulaire (9).

5. Le kit selon la revendication 4, dans lequel l'extrémité distale du tube interne (5) du cathéter (10) du second dispositif médical est en communication à fluide avec la zone imperméable aux liquides de l'élément tubulaire (9) et l'extrémité proximale du tube interne (5) est en communication à fluide avec l'entrée du dispositif de séparation.

6. Le kit selon la revendication 2, dans lequel le transporteur du second dispositif médical est composé de treillis métallique tressé.

7. Le kit selon l'une quelconque des revendications 1-6, dans lequel le dispositif de séparation (25) comprend au moins un filtre muni d'ouvertures dont la taille est comprise entre 15 et 65 micromètres, de préférence entre 20 et 30 micromètres, plus préférentiellement environ 25 micromètres.

8. Le kit selon l'une quelconque des revendications 1-7, dans lequel le dispositif de séparation (25) comprend au moins deux filtres, un premier filtre et un second filtre.

9. Le kit selon l'une quelconque des revendications 1-8, dans lequel les ouvertures du premier filtre sont au moins 15% plus grandes que les ouvertures du second filtre à travers lequel le sang coulera.

10. Le kit selon l'une quelconque des revendications 1-9, comprenant au moins une pompe appropriée à être connectée à l'extrémité proximale du cathéter du second dispositif médical récupérable (10)

11. Le kit selon l'une quelconque des revendications 1-10, comprenant au moins un troisième dispositif médical récupérable pour l'isolement et/ou la perfusion des veines, ledit troisième dispositif médical comprend une lumière (58), une lumière de gonflage (61), un ballon (59) à l'extrémité distale (X) du dispositif et une pluralité de sorties (62), lesdites sorties (62) sont positionnées à l'extrémité distale (X) du ballon (59) ou à l'extrémité proximale (Y) du ballon (59).

12. Le kit selon l'une quelconque des revendications 1-11, dans lequel le premier dispositif médical récupérable comprend au moins un ballon de gonflage (65), ledit ballon (65) est gonflé en coulant de l'agent thérapeutique dans la lumière du dispositif et ouvertures profondes (68, 69) munies dans ledit dispositif.
